# EUROPÄISCHE PATENTSCHRIFT

(11) **EP 3 157 901 B1**
(45) Veröffentlichungstag und Bekanntmachung des Hinweises auf die Patenterteilung: **21.03.2018**
(21) Anmeldenummer: 15727682.5
(22) Anmeldetag: 12.06.2015
(51) Int. Cl.: C07C 227/18, C07C 227/36, C07C 229/16

(54) **FORMULIERUNGEN, IHRE HERSTELLUNG UND VERWENDUNG, UND GEEIGNETE KOMPONENTEN**
FORMULATIONS, THE PRODUCTION AND USE THEREOF, AND SUITABLE COMPONENTS
MÉLANGES D'ÉNANTIOMÈRES ET PROCÉDÉ DE FABRICATION DE TELS MÉLANGES

(30) Priorität: 23.06.2014 EP 14173388
(43) Veröffentlichungstag der Anmeldung: 26.04.2017
(73) Patentinhaber: BASF SE, 67056 Ludwigshafen am Rhein (DE)
(72) Erfinder: TÜRK, Holger, 68161 Mannheim (DE); WEBER, Heike, 68239 Mannheim (DE); TUERKOGLU, Gazi, 68163 Mannheim (DE)
(74) Vertreter: BASF IP Association
(86) Internationale Anmeldenummer: PCT/EP2015/063134
(87) Internationale Veröffentlichungsnummer: WO 2015/197379

(56) Entgegenhaltungen:
- EP-A1- 2 138 560
- WO-A1-2013/056996

## Beschreibung

Die vorliegende Anmeldung betrifft Formulierungen, enthaltend
(A) mindestens eine Verbindung, gewählt aus Methylglycindiacetat (MGDA) und Glutaminsäurediacetat (GLDA) sowie deren Salzen,
(B) mindestens ein Pfropfcopolymer, aufgebaut aus
   (a) mindestens einer Pfropfgrundlage, gewählt aus nicht-ionischen Monosacchariden, Disacchariden, Oligosacchariden und Polysacchariden,
      und Seitenketten, erhältlich durch Aufpfropfen von
   (b) mindestens einer ethylenisch ungesättigten Mono- oder Dicarbonsäure und
   (c) mindestens einer Verbindung der allgemeinen Formel (I), 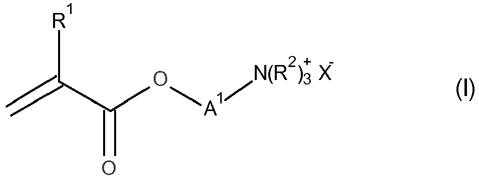
   wobei die Variablen wie folgt definiert sind:
   R¹ ist gewählt aus Methyl und Wasserstoff,
   A¹ ist gewählt aus C₂-C₄-Alkylen,
   R² sind gleich oder verschieden und gewählt aus C₁-C₄-Alkyl,
   X- ist gewählt aus Halogenid, Mono-C₁-C₄-Alkylsulfat und Sulfat.

Weiterhin betrifft die vorliegende Anmeldung Verwendungen der erfindungsgemäßen Formulierungen und ein Verfahren zu ihrer Herstellung. Weiterhin betrifft die vorliegenden Erfindung Pfropfcopolymere, die aufgebaut sind aus
(a) mindestens einer Pfropfgrundlage, gewählt aus nicht-ionischen Monosacchariden, Disacchariden, Oligosacchariden und Polysacchariden,
   und Seitenketten, erhältlich durch Aufpfropfen von
(b) mindestens einer ethylenisch ungesättigten Mono- oder Dicarbonsäure und
(c) mindestens einer Verbindung der allgemeinen Formel (I), 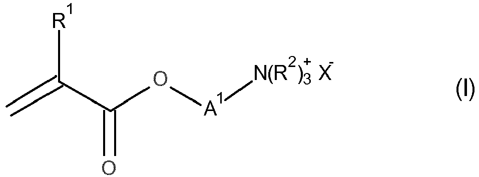 wobei die Variablen wie folgt definiert sind:
   R¹ ist gewählt aus Methyl und Wasserstoff,
   A¹ ist gewählt aus C₂-C₄-Alkylen,
   R² sind gleich oder verschieden und gewählt aus C₁-C₄-Alkyl,
   X⁻ ist gewählt aus Halogenid, Mono-C₁-C₄-Alkylsulfat und Sulfat.

Geschirrspülmittel haben vielerlei Anforderungen zu erfüllen. So haben sie das Geschirr gründlich zu reinigen, sie sollen im Abwasser keine schädlichen oder potenziell schädlichen Substanzen aufweisen, sie sollen das Ablaufen und Trocknen des Wassers vom Geschirr gestatten, die abgelösten Schmutzbestandteile müssen so nachhaltig dispergiert oder emulgiert werden, dass sie sich nicht auf der Oberfläche des Spülguts ablagern. Die Geschirrspülmittel sollen beim Betrieb der Spülmaschine nicht zu Problemen führen. Schließlich sollen sie nicht zu ästhetisch unerwünschten Folgen am zu reinigenden Gut führen. Insbesondere sollen keine weißlichen Flecken oder Beläge auftreten, die aufgrund der Anwesenheit von Kalk oder anderen anorganischen und organischen Salzen bei der Eintrocknung von Wassertropfen entstehen oder durch Ablagerung von Schmutzbestandteilen oder anorganischen Salzen sich schon während des Spülvorgangs auf dem Spülgut niederschlagen.

Insbesondere in modernen maschinellen Geschirrreinigern, den multifunktionellen Reinigern (z.B. 3-in-1-Reinigern oder allgemein x-in-1-Reinigern), sind die Funktionen des Reinigens, des Klarspülens und der Wasserenthärtung in einer einzigen Reinigerformulierung vereint, so dass für den Verbraucher sowohl das Nachfüllen von Salz (bei Wasserhärten von 0° bis 21° dH) als auch das Nachfüllen von Klarspülmittel überflüssig wird.

In x-in-1-Reinigern werden häufig Polymere zur Belagsinhibierung eingesetzt. Dies können in phosphathaltigen Reinigern beispielsweise sulfonathaltige Polymere sein, die insbesondere Effekte auf die Inhibierung von Calciumphosphatniederschlägen zeigen. Die eingesetzten Tenside sind so gewählt, dass sie in den Klarspülgang verschleppt werden und dort für die optimale Benetzung und ein gutes Klarspülergebnis sorgen. Weitere geeignete Polymere sind Polycarboxylate wie beispielsweise Polyacrylsäuren.

Der Trend zu phosphatfreien Reinigungsmitteln, die auch weiterhin ohne Klarspüler und lonentauscher eingesetzt werden sollen, erfordert jedoch neue Lösungen. In phosphatfreien Geschirrreinigungsmitteln ist die Zusammensetzung der anfallenden Salze eine andere als in phosphathaltigen Reinigern, so dass bisher verwendete Polymere in vielen Fällen nicht ausreichend wirksam sind. Insbesondere was die Belagsinhibierung betrifft, sind phosphatfreie Geschirrreinigungsmittel noch verbesserungsbedürftig.

In EP 2 138 560 A1 werden Pfropfcopolymere und ihre Verwendung in Mitteln zur Reinigung harter Oberflächen offenbart, unter anderem als Geschirrreiniger. Die in EP 2 138 560 A1 vorgeschlagenen Reinigungsmittel weisen jedoch in manchen Fällen, beispielsweise als Geschirrreinigungsmittel auf Besteckteilen wie Messern und insbesondere auf Glas, keine ausreichende Belagsinhibierung auf.

Es bestand daher die Aufgabe, Formulierungen bereit zu stellen, die eine sehr gute Belagsinhibierung - insbesondere in Phosphat-freien Mitteln -insbesondere auf Glas aufweisen. Es bestand weiterhin die Aufgabe, ein Verfahren bereit zu stellen, durch das Formulierungen hergestellt werden können, die eine sehr gute Belagsinhibierung - insbesondere in Phosphat-freien Mitteln - aufweisen. Es bestand schließlich die Aufgabe, geeignete Komponenten für derartige Formulierungen bereit zu stellen.

Dem entsprechend wurden die eingangs definierten Formulierungen gefunden, im Rahmen der vorliegenden Erfindung auch erfindungsgemäße Formulierungen genannt.

Erfindungsgemäße Formulierungen können bei Zimmertemperatur, also bei 20°C, flüssig, fest, pastenförmig oder gelförmig sein. Vorzugsweise sind erfindungsgemäße Formulierungen bei Zimmertemperatur fest. Bei Zimmertemperatur feste erfindungsgemäße Formulierungen können wasserfrei sein oder Wasser enthalten, beispielsweise bis zu 20 Gew.-%, bevorzugt 0,1 bis 10 Gew.-% Wasser, bestimmbar beispielsweise durch Karl-Fischer-Titration oder durch Bestimmung des Trockenrückstands bei 80°C unter vermindertem Druck. Bei Zimmertemperatur feste erfindungsgemäße Formulierungen können beispielsweise in Form von Pulver, Granulat oder Tabletten vorliegen.

In einer anderen Ausführungsform sind erfindungsgemäße Formulierungen bei 20°C flüssig. Bei 20°C flüssige erfindungsgemäße Formulierungen können 30 bis 80 Gew.-% Wasser enthalten, bevorzugt 40 bis 80 Gew.-%. Auch in solchen Ausführungsformen kann der Wassergehalt durch Bestimmung des Trockenrückstands bei 80°C unter vermindertem Druck bestimmt werden. Bei Zimmertemperatur flüssige erfindungsgemäße Formulierungen können beispielsweise in Gelform vorliegen.

Erfindungsgemäße Formulierungen enthalten
(A) mindestens eine Verbindung, kurz auch Verbindung (A) genannt, gewählt aus Methylglycindiacetat (MGDA) und Glutaminsäurediacetat (GLDA) sowie deren Salzen. Bevorzugt wählt man Verbindung (A) aus MGDA und seinen Salzen, insbesondere seinen Natriumsalzen.

MGDA und GLDA können als Racemat vorliegen oder als enantiomerenreine Verbindung. GLDA wählt man bevorzugt aus L-GLDA oder enantiomerenangereicherten Mischungen von L-GLDA, in denen mindestens 80 mol-%, bevorzugt mindestens 90 mol-% L-GLDA vorliegt.

In einer Ausführungsform der vorliegenden Erfindung wählt man Verbindung (A) aus racemischem MGDA. In einer anderen Ausführungsform der vorliegenden Erfindung wählt man Verbindung (A) aus L-MGDA oder aus Enantiomerengemischen von L-und D-MGDA, in denen L-MGDA überwiegt und in denen das Molverhältnis L/D im Bereich von 55:45 bis 95:5 ist, bevorzugt 60:40 bis 85:15. Das Molverhältnis L/D kann man beispielsweise durch Polarimetrie bestimmen oder chromatographisch, bevorzugt durch HPLC mit einer chiralen Säule, beispielsweise mit Cyclodextrin als stationärer Phase oder mit einem auf der Säule immobilisierten optisch aktiven Ammoniumsalz. Beispielsweise kann man ein immobilisiertes D-Penicillaminsalz einsetzen.

MGDA bzw. GLDA setzt man vorzugsweise als Salz ein. Bevorzugte Salze sind Ammoniumsalze und Alkalimetallsalze, besonders bevorzugt sind die kalium- und insbesondere die Natriumsalze. Diese können beispielsweise die allgemeine Formel (II) bzw. (III) aufweisen:

[CH₃-CH(COO)-N(CH₂-COO)₂]Na_{3-x-y}KₓH_{y} (II)

- x: im Bereich von 0.0 bis 0.5, bevorzugt bis 0.25,
- y: im Bereich von 0.0 bis 0.5, bevorzugt bis 0.25.

[OOC-(CH₂)₂-CH(COO)-N(CH₂-COO)₂]Na_{4-x-y}KₓH_{y} (III)
- x: im Bereich von 0.0 bis 0.5, bevorzugt bis 0.25,
- y: im Bereich von 0.0 bis 0.5, bevorzugt bis 0.25.

Ganz besonders bevorzugt sind das Trinatriumsalz von MGDA und das Tetranatriumsalz von GLDA.

Verbindung (A) kann in geringen Mengen Kationen enthalten, die von Alkalimetallionen verschieden sind, beispielsweise Mg²⁺, Ca²⁺ oder Eisenionen, beispielsweise Fe²⁺ oder Fe³⁺. Derartige Ionen sind in vielen Fällen herstellungsbedingt in Verbindung (A) enthalten. Von Alkalimetallionen verschiedene Kationen sind in einer Ausführungsform der vorliegenden Erfindung im Bereich von 0.01 bis 5 mol-% enthalten, bezogen auf gesamtes MGDA bzw. gesamtes GLDA.

In einer anderen Ausführungsform der vorliegenden Erfindung sind keine messbaren Anteile an Kationen, die von Alkalimetallionen verschieden sind, in Verbindung (A) enthalten.

In einer Ausführungsform der vorliegenden Erfindung enthält Verbindung (A) geringe Mengen einer oder mehrerer Verunreinigungen, die herstellungsbedingt sein kann bzw. können. Im Falle von MGDA kann als Verunreinigung beispielsweise Propionsäure, Alanin oder Milchsäure enthalten sein. Geringe Mengen sind dabei Anteile beispielsweise im Bereich von 0,01 bis 1 Gew.-%, bezogen auf Verbindung (A). Derartige Verunreinigungen werden im Rahmen der vorliegenden Erfindung vernachlässigt, wenn nicht ausdrücklich anders angegeben.

In einer Ausführungsform der vorliegenden Erfindung enthält erfindungsgemäße Formulierung eine Verbindung (A), beispielsweise nur Trinatriumsalz von MGDA oder nur Tetranatriumsalz von GLDA. Dabei sollen auch Verbindungen der Formel (II) bzw. (III) mit x oder y ungleich null jeweils als eine Verbindung bezeichnet werden.

In einer anderen Ausführungsform der vorliegenden Erfindung enthält erfindungsgemäße Formulierung zwei Verbindungen (A), beispielsweise eine Mischung von Trinatriumsalz von MGDA und Tetranatriumsalz von GLDA, beispielsweise in einem Molverhältnis im Bereich von 1:1 bis 1:10.

Erfindungsgemäße Formulierungen enthalten weiterhin
(B) mindestens ein Pfropfcopolymer, das im Rahmen der vorliegenden Erfindung auch Pfropfcopolymer (B) oder erfindungsgemäße Pfropfcopolymer (B) genannt wird und das aufgebaut ist aus
   (a) mindestens einer Pfropfgrundlage, kurz Pfropfgrundlage (a) genannt, die gewählt ist aus nicht-ionischen Monosacchariden, Disacchariden, Oligosacchariden und Polysacchariden,
   und Seitenketten, erhältlich durch Aufpfropfen von
   (b) mindestens einer ethylenisch ungesättigten Mono- oder Dicarbonsäure, kurz Monocarbonsäure (b) bzw. Dicarbonsäure (b) genannt, und
   (c) mindestens einer Verbindung der allgemeinen Formel (I), kurz Monomer (c) oder Verbindung (I) genannt, 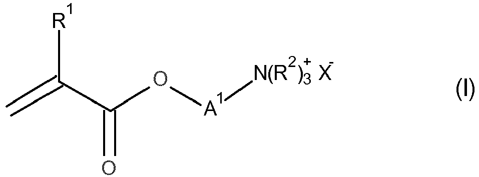
   wobei die Variablen wie folgt definiert sind:
   R¹ ist gewählt aus Methyl und Wasserstoff,
   A¹ ist gewählt aus C₂-C₄-Alkylen,
   R² sind gleich oder verschieden und gewählt aus C₁-C₄-Alkyl,
   X⁻ ist gewählt aus Halogenid, Mono-C₁-C₄-Alkylsulfat und Sulfat.

Als Pfropfgrundlage (a) geeignete nicht-ionische Monosaccharide kann man beispielsweise Aldopentosen, Pentulosen (Ketopentosen), Aldohexosen und Hexulosen (Ketohexosen) wählen. Geeignete Aldopentosen sind z.B. D-Ribose, D-Xylose und L-Arabinose. Als Aldohexosen seien D-Glucose, D-Mannose und D-Galactose genannt; als Beispiele von Hexulosen (Ketohexosen) sind vor allem D-Fructose und D-Sorbose zu nennen.

Im Rahmen der vorliegenden Erfindung sollen auch Desoxyzucker wie beispielsweise L-Fucose und L-Rhamnose zu nicht-ionischen Monosacchariden gezählt werden.

Als Beispiele für nicht-ionische Disaccharide seien beispielsweise Cellobiose, Lactose, Maltose und Saccharose genannt.

Als nicht-ionische Oligosaccharide seien im Rahmen der vorliegenden Erfindung nicht-ionische Kohlenhydrate mit drei bis zehn nicht-ionischen Monosaccharideinheiten pro Molekül bezeichnet, beispielsweise Glycane. Als nicht-ionische Polysaccharide werden im Rahmen der vorliegenden Erfindung nicht-ionische Kohlenhydrate mit mehr als zehn nicht-ionischen Monosaccharideinheiten pro Molekül bezeichnet. Nicht-ionische Oligo- und Polysaccharide können beispielsweise linear, cyclisch oder verzweigt sein.

Als nicht-ionische Polysaccharide beispielhaft zu nennen sind Biopolymere wie Stärke und Glycogen sowie Cellulose und Dextran. Weiterhin zu nennen sind Inulin als Polykondensat der D-Fructose (Fructane) und Chitin. Weitere Beispiele von nicht-ionischen Polysacchariden sind nicht-ionische Stärkeabbauprodukte, beispielsweise Produkte, die man durch enzymatischen oder sogenannten chemischen Abbau von Stärke erhalten kann. Ein Beispiel für den sogenannten chemischen Abbau von Stärke ist die säurekatalysierte Hydrolyse.

Bevorzugte Beispiele für nicht-ionische Stärkeabbauprodukte sind Maltodextrine. Unter Maltodextrin werden im Rahmen der vorliegenden Erfindung Gemische aus Monomeren, Dimeren, Oligomeren und Polymeren der Glucose gefasst. Je nach Hydrolysegrad unterscheidet sich die prozentuale Zusammensetzung. Diese wird durch das Dextrose-Äquivalent beschrieben, das bei Maltodextrin zwischen 3 und 40 liegt.

Bevorzugt wählt man Pfropfgrundlage (a) aus nicht-ionischen Polysacchariden, insbesondere aus Stärke, die vorzugsweise nicht chemisch modifiziert ist, beispielsweise deren Hydroxylgruppen vorzugsweise weder verestert noch verethert sind. In einer Ausführungsform der vorliegenden Erfindung wählt man Stärke aus solchen nicht-ionischen Polysacchariden, die im Bereich von 20 bis 30 Gew.-% Amylose und im Bereich von 70 bis 80% Amylopektin aufweisen. Beispiele sind Maisstärke, Reisstärke, Kartoffelstärke und Weizenstärke.

Auf die Pfropfgrundlage (a) sind Seitenketten aufgepfropft. Pro Molekül von Pfropfcopolymer (B) können vorzugsweise im Mittel eine bis zehn Seitenketten aufgepfropft sein. Vorzugsweise ist dabei eine Seitenkette mit dem anomeren C-Atom eines Monosaccharids oder mit einem anomeren C-Atom des Kettenendes eines Oligo- oder Polysaccharids verknüpft. Die Zahl der Seitenketten ist durch die Zahl der C-Atome mit Hydroxyl-Gruppen der betreffenden Pfropfgrundlage (a) nach oben begrenzt.

Beispiele für Monocarbonsäuren (b) sind ethylenisch ungesättigte C₃-C₁₀-Monocarbonsäuren und deren Alkalimetall- oder Ammoniumsalze, insbesondere die Kalium- und die Natriumsalze. Bevorzugte Monocarbonsäuren (b) sind die Acrylsäure und die Methacrylsäure sowie Natrium(meth)acrylat. Auch Mischungen von ethylenisch ungesättigten C₃-C₁₀ Monocarbonsäuren und insbesondere Mischungen von Acryl- und Methacrylsäure sind bevorzugte Komponenten (b).

Beispiele für Dicarbonsäuren (b) sind ethylenisch ungesättigte C₄-C₁₀-Dicarbonsäuren und deren Mono- und insbesondere Dialkalimetall- oder Ammoniumsalze, insbesondere die Dikalium- und die Dinatriumsalze, sowie Anhydride von ethylenisch ungesättigten C₄-C₁₀-Dicarbonsäuren. Bevorzugte Dicarbonsäuren (b) sind die Maleinsäure, Fumarsäure, Itaconsäure sowie Maleinsäureanhydrid und Itaconsäureanhydrid.

In einer Ausführungsform enthält Pfropfcopolymer (B) in mindestens einer Seitenkette neben Monomer (c) mindestens eine Monocarbonsäure (b) und mindestens eine Dicarbonsäure (b). In einer bevorzugten Ausführungsform der vorliegenden Erfindung enthält Pfropfcopolymer (B) in den Seitenketten neben Monomer (c) ausschließlich Monocarbonsäure (b), aber keine Dicarbonsäure (b) einpolymerisiert.

Monomere (c) sind ethylenisch ungesättigte N-haltige Verbindungen mit permanenter kationischer Ladung. 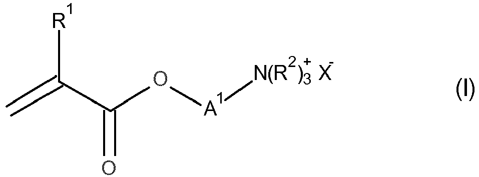 wobei die Variablen wie folgt definiert sind:
R¹ ist gewählt aus Methyl und Wasserstoff,
A¹ ist gewählt aus C₂-C₄-Alkylen, beispielsweise -CH₂-CH₂-, CH₂-CH(CH₃)-, -(CH₂)₃-, -(CH₂)₄-, bevorzugt sind -CH₂-CH₂- und -(CH₂)₃-,
R² sind verschieden oder vorzugsweise gleich und gewählt aus C₁-C₄-Alkyl, beispielsweise Methyl, Ethyl, n-Propyl, n-Butyl, iso-Propyl, iso-Butyl, sec.-Butyl, tert.-Butyl, bevorzugt sind mindestens zwei R² gleich und jeweils Methyl, und die dritte Gruppe R² ist Ethyl, n-Propyl oder n-Butyl, oder zwei R² sind gleich und jeweils Ethyl, und die dritte Gruppe R² ist Methyl, n-Propyl oder n-Butyl. Besonders bevorzugt sind alle drei R² jeweils gleich und gewählt aus Methyl.
X⁻ ist gewählt aus Halogenid, beispielsweise lodid, Bromid und insbesondere Chlorid, weiterhin aus Mono-C₁-C₄-Alkylsulfat und Sulfat. Beispiele für Mono-C₁-C₄-Alkylsulfat sind Methylsulfat, Ethylsulfat, iso-Propylsulfat und n-Butylsulfat, bevorzugt sind Methylsulfat und Ethylsulfat. Wenn man X- als Sulfat wählt, so steht X- für ein halbes Äquivalent Sulfat.

In einer bevorzugten Ausführungsform der vorliegenden Erfindung sind in Monomer (c) die Variablen wie folgt gewählt:
R¹ ist Wasserstoff oder Methyl,
R² sind gleich und jeweils Methyl,
A¹ ist CH₂CH₂, und
X- ist Chlorid.

In einer Ausführungsform der vorliegenden Erfindung ist Monomer (c) gewählt aus

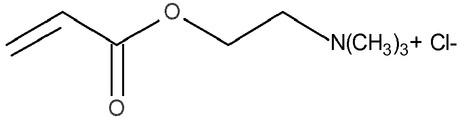

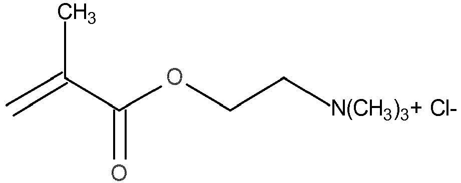

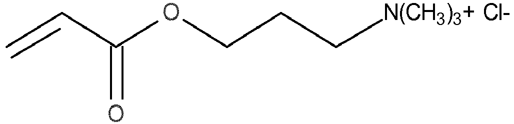

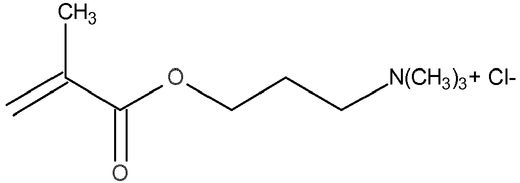

Pfropfcopolymer (B) kann in einer oder mehreren Seitenketten mindestens ein weiteres Comonomer (d) einpolymerisiert enthalten, beispielsweise Hydroxyalkylester wie 2-Hydroxyethyl-(meth)acrylat oder 3-Hydroxypropyl(meth)acrylat, oder Ester von alkoxylierten Fettalkoholen, oder Sulfonsäuregruppen-haltige Comonomere, beispielsweise 2-Acrylamido-2-methylpropansulfonsäure (AMPS) und ihre Alkalimetallsalze.

Vorzugsweise enthält Pfropfcopolymer (B) außer Monomer (c) und Monocarbonsäure (b) bzw. Dicarbonsäure (b) keine weiteren Comonomere (d) in einer oder mehreren Seitenketten.

In einer Ausführungsform der vorliegenden Erfindung ist der Anteil von Pfropfgrundlage (a) in Pfropfcopolymer (B) im Bereich von 40 bis 95 Gew.-%, vorzugsweise von 50 bis 90 Gew.-%, jeweils bezogen auf gesamtes Pfropfcopolymer (B).

In einer Ausführungsform der vorliegenden Erfindung ist der Anteil von Monocarbonsäure (b) bzw. Dicarbonsäure (b) im Bereich von 2 bis 40 Gew.-%, vorzugsweise von 5 bis 30 Gew.-% und insbesondere von 5 bis 25 Gew.-%, jeweils bezogen auf gesamtes Pfropfcopolymer (B).

Monomer bzw. Monomere vom Typ (c) ist bzw. sind in Mengen von 5 bis 50 Gew.-%, vorzugsweise von 5 bis 40 Gew.-% und besonders bevorzugt von 5 bis 30 Gew.-% einpolymerisiert, jeweils bezogen auf gesamtes Pfropfcopolymer (B).

Es ist bevorzugt, wenn Pfropfcopolymer (B) mehr Monocarbonsäure (b) als Verbindung (c) einpolymerisiert enthält, und zwar auf die molaren Anteile bezogen, beispielsweise im Bereich von 1,1:1 bis 5:1, bevorzugt 2:1 bis 4:1.

In einer Ausführungsform der vorliegenden Erfindung ist das mittlere Molekulargewicht (M_{w}) von Pfropfcopolymer (B) im Bereich von 2.000 bis 200.000 g/mol, vorzugsweise von 5.000 bis 150.000 und insbesondere im Bereich von 8.000 bis 100.000 g/mol. Das mittlere Molekulargewicht M_{w} misst man vorzugsweise durch Gelpermeationschromatographie in wässriger KCI/Ameisensäure-Lösung.

Pfropfcopolymer (B) kann man vorzugsweise als wässrige Lösung erhalten, aus der man es isolieren kann, z. B. durch Sprühtrocknung, Sprühgranulierung oder Gefriertrocknung. Wahlweise kann man Lösung von Pfropfcopolymer (B) oder getrocknetes Pfropfcopolymer (B) zur Herstellung der erfindungsgemäßen Formulierungen verwenden.

Es ist bevorzugt, Pfropfcopolymer (B) durch mindestens ein Biozid zu stabilisieren. Beispiele für geeignete Biozide sind Isothiazolinone, beispielsweise 1,2-Benzisothiazolin-3-on ("BIT"), Octylisothiazolinon ("OIT"), Dichloroktylisothiazolinon ("DCOIT"), 2-Methyl-2*H*-isothiazolin-3-on ("MIT") und 5-Chlor-2-methyl-2*H-*isothiazolin-3-one("CIT"), Phenoxyethanol, Alkylparabene wie Methylparaben, Ethylparaben, Propylparaben, Benzoesäure und ihre Salze wie z.B. Natriumbenzoat, Benzylalkohol, Alkalimetallsorbate wie z.B. Natriumsorbat, und gegebenenfalls substituierte Hydantoine wie z.B. 1,3-Bis(hydroxymethyl)-5,5-dimethylhydantoin (DMDM-Hydantoin). Weitere Beispiele sind 1,2-Dibrom-2, 4-dicyanobutan, lodo-2-propynyl-butyl-carbamat, lod und lodophore.

In einer Ausführungsform der vorliegenden Erfindung ist erfindungsgemäße Formulierung frei von Phosphaten und Polyphosphaten, wobei Hydrogenphosphate mit subsumiert sind, beispielsweise frei von Trinatriumphosphat, Pentanatriumtripolyphosphat und Hexanatriummetaphosphat. Unter "frei von" soll im Zusammenhang mit Phosphaten und Polyphosphaten im Rahmen der vorliegenden Erfindung verstanden werden, dass der Gehalt an Phosphat und Polyphosphat in Summe im Bereich von 10 ppm bis 0,2 Gew.-% liegt, bestimmt durch Gravimetrie.

In einer Ausführungsform der vorliegenden Erfindung ist erfindungsgemäße Formulierung frei von solchen Schwermetallverbindungen, die nicht als Bleichkatalysatoren wirken, insbesondere von Verbindungen des Eisens. Unter "frei von" soll im Zusammenhang mit Schwermetallverbindungen im Rahmen der vorliegenden Erfindung verstanden werden, dass der Gehalt an Schwermetallverbindungen, die nicht als Bleichkatalysatoren wirken, in Summe im Bereich von 0 bis 100 ppm liegt, bevorzugt 1 bis 30 ppm, bestimmt nach der Leach-Methode.

Als "Schwermetalle" gelten im Rahmen der vorliegenden Erfindung alle Metalle mit einer spezifischen Dichte von mindestens 6 g/cm³, mit der Ausnahme von Zink und Wismut. Insbesondere gelten als Schwermetalle Edelmetalle sowie Eisen, Kupfer, Blei, Zinn, Nickel, Cadmium und Chrom.

In einer Ausführungsform der vorliegenden Erfindung enthält erfindungsgemäße Formulierung
insgesamt im Bereich von 1 bis 50 Gew.-% Verbindung (A), bevorzugt 5 bis 45 Gew.-%, besonders bevorzugt 10 bis 35 Gew.-%;
insgesamt im Bereich von 0,1 bis 4 Gew.-% Pfropfcopolymer (B), bevorzugt 0,3 bis 2 Gew.-%, besonders bevorzugt 0,5 bis 1,5 Gew.-%,
bezogen jeweils auf Feststoffgehalt der betreffenden erfindungsgemäßen Formulierung.

Erfindungsgemäße Formulierungen können frei sein von Bleichmitteln, beispielsweise frei von anorganischen Peroxidverbindungen oder Chlorbleichmitteln wie Natriumhypochlorit. Unter frei von anorganischen Peroxidverbindungen oder Chlorbleichmitteln soll dabei verstanden werden, dass derartige erfindungsgemäße Formulierungen insgesamt 0,01 Gew.-% oder weniger anorganische Peroxidverbindung und Chlorbleichmittel enthalten, bezogen jeweils auf Feststoffgehalt der betreffenden erfindungsgemäßen Formulierung.

In einer anderen Ausführungsform der vorliegenden Erfindung enthält erfindungsgemäße Formulierung
(C) mindestens eine anorganische Peroxidverbindung, im Rahmen der vorliegenden Erfindung kurz auch als Peroxid (C) bezeichnet wird. Peroxid (C) ist gewählt aus Natriumperoxodisulfat, Natriumperborat und Natriumpercarbonat, bevorzugt ist Natriumpercarbonat.

Peroxid (C) kann wasserfrei oder vorzugsweise wasserhaltig sein. Beispiele für wasserhaltiges Natriumperborat ist Na₂[B(OH)₂(O₂)]₂), manchmal auch als NaBO₂·O₂·3H₂O geschrieben wird.

Beispiel für wasserhaltiges Natriumpercarbonat ist 2 Na₂CO₃·3 H₂O₂. Besonders bevorzugt wählt man Peroxid (C) aus wasserhaltigen Percarbonaten.

Bevorzugt enthält erfindungsgemäße Formulierung im Bereich von 1 bis 20 Gew.-% Peroxid (C), bevorzugt 2 bis 12 Gew.-%, besonders bevorzugt 3 bis 12 Gew.-%, bezogen auf Feststoffgehalt der betreffenden Formulierung.

Erfindungsgemäße Formulierungen, die mindestens ein Peroxid (C) enthalten, sind bei Zimmertemperatur vorzugsweise fest.

In einer anderen Ausführungsform enthält erfindungsgemäße Formulierung
(C) mindestens ein Chlor-haltiges Bleichmittel, welches im Rahmen der vorliegenden Erfindung kurz auch als Chlorbleichmittel (C) bezeichnet wird. Bei Chlorbleichmittel (C) handelt es sich vorzugsweise um Natriumhypochlorit.

Chlorbleichmittel-(C)-haltige erfindungsgemäße Formulierungen sind bei Zimmertemperatur vorzugsweise flüssig.

Bevorzugt enthält erfindungsgemäße Formulierung im Bereich von 0,1 bis 20 Gew.-% Chlorbleichmittel (C), bevorzugt 0,5 bis 12 Gew.-%, besonders bevorzugt 1 bis 12 Gew.-%, bezogen auf Feststoffgehalt der betreffenden flüssigen Formulierung.

Erfindungsgemäße Formulierungen können einen oder mehrere weitere Inhaltsstoffe (D) enthalten. Inhaltsstoffe (D) sind von Verbindung (A), Pfropfcopolymer (B) und Peroxid (C) bzw. Chlorbleichmittel (C) verschieden.

Erfindungsgemäße Formulierungen können einen oder mehrere weitere Inhaltsstoffe (D) aufweisen, beispielsweise ein oder mehrere Tenside, ein oder mehrere Enzyme, einen oder mehrere Enzymstabilisatoren, einen oder mehrere Builder, insbesondere Phosphat-freie Builder, einen oder mehrere Cobuilder, einen oder mehrere Alkaliträger, eine oder mehrere Säuren, einen oder mehrere Bleichkatalysatoren, einen oder mehrere Bleichaktivatoren, einen oder mehrere Bleichmittelstabilisatoren, einen oder mehrere Entschäumer, einen oder mehrere Korrosionsinhibitoren, einen oder mehrere Gerüststoffe, Puffer, Farbstoffe, einen oder mehrere Duftstoffe, ein oder mehrere Verdickungsmittel, ein oder mehrere organische Lösungsmittel, ein oder mehrere Tablettierhilfsmittel, ein oder mehrere Disintegrationsmittel, auch Tablettensprengmittel genannt, oder einen oder mehrere Löslichkeitsvermittler.

Beispiele für Tenside sind insbesondere nicht-ionische Tenside sowie Mischungen von anionischen oder zwitterionischen Tensiden mit nicht-ionischen Tensiden. Bevorzugte nicht-ionische Tenside sind alkoxylierte Alkohole und alkoxylierte Fettalkohole, Di- und Multiblockcopolymerisate von Ethylenoxid und Propylenoxid und Umsetzungsprodukte von Sorbitan mit Ethylenoxid oder Propylenoxid, Alkylglycoside und sogenannte Aminoxide.

Bevorzugte Beispiele für alkoxylierte Alkohole und alkoxylierte Fettalkohole sind Verbindungen der allgemeinen Formel (IV) 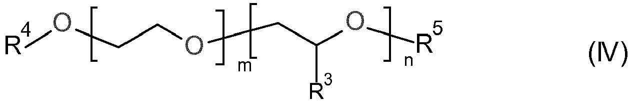 in der die Variablen wie folgt definiert sind:
R³ gleich oder verschieden gewählt aus linearem C₁-C₁₀-Alkyl, bevorzugt Ethyl und besonders bevorzugt Methyl,
R⁴ gewählt aus C₈-C₂₂-Alkyl, beispielsweise n-C₈H₁₇, n-C₁₀H₂₁, n-C₁₂H₂₅, n-C₁₄H₂₉, n-C₁₆H₃₃ oder n-C₁₈H₃₇,
R⁵ gewählt aus Wasserstoff und C₁-C₁₀-Alkyl, Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl oder iso-Decyl,

m und n liegen im Bereich von null bis 300, wobei die Summe von n und m mindestens eins beträgt. Bevorzugt ist m im Bereich von 1 bis 100 und n im Bereich von 0 bis 30.

Dabei kann es sich bei Verbindungen der allgemeinen Formel (IV) um Blockcopolymere oder statistische Copolymere handeln, bevorzugt sind Blockcopolymere.

Andere bevorzugte Beispiele für alkoxylierte Alkohole und alkoxylierte Fettalkohole sind Verbindungen der allgemeinen Formel (V) 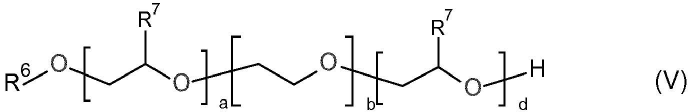 in der die Variablen wie folgt definiert sind:
- R⁶: gewählt aus C₆-C₂₀-Alkyl, insbesondere n-C₈H₁₇, n-C₁₀H₂₁, n-C₁₂H₂₅, n-C₁₄H₂₉, n-C₁₆H₃₃, n-C₁₈H₃₇,
- R⁷: gleich oder verschieden und gewählt aus linearem C₁-C₄-Alkyl, bevorzugt jeweils gleich und Ethyl und besonders bevorzugt Methyl.
- a: eine Zahl im Bereich von 1 bis 6,
- b: ist eine Zahl im Bereich von 4 bis 20,
- d: ist eine Zahl im Bereich von 4 bis 25.

Dabei kann es sich bei Verbindungen der allgemeinen Formel (V) um Blockcopolymere oder statistische Copolymere handeln, bevorzugt sind Blockcopolymere.

Andere bevorzugte Beispiele für alkoxylierte Alkohole und alkoxylierte Fettalkohole sind Hydroxymischether der allgemeinen Formel (VI)

R⁸-CH(OH)-CH₂-O-(AO)ₖ-R⁹ (VI)

wobei die Variablen wie folgt gewählt werden:
- R⁸: C₄-C₃₀-Alkyl, verzweigt oder unverzweigt, oder C₄-C₃₀-Alkenyl, verzweigt oder unverzweigt, mit mindestens einer C-C-Doppelbindung.

Bevorzugt ist R⁸ gewählt aus C₄-C₃₀-Alkyl, verzweigt oder unverzweigt, besonders bevorzugt unverzweigtes C₄-C₃₀-Alkyl und ganz besonders bevorzugt n-C₁₀-C₁₂-Alkyl.
- R⁹: C₁-C₃₀-Alkyl, verzweigt oder unverzweigt, oder, C₂-C₃₀-Alkenyl, verzweigt oder unverzweigt, mit mindestens einer C-C-Doppelbindung.

Bevorzugt ist R⁹ gewählt aus C₄-C₃₀-Alkyl, verzweigt oder unverzweigt, besonders bevorzugt unverzweigtes C₆-C₂₀-Alkyl und ganz besonders bevorzugt n-C₈-C₁₁-Alkyl.
- k: ist eine Zahl im Bereich von 1 bis 100, bevorzugt von 5 bis 60, besonders bevorzugt 10 bis 50 und ganz besonders bevorzugt 20 bis 40,
- AO: ist gewählt aus Alkylenoxid, verschieden oder gleich und gewählt aus CH₂-CH₂-O, (CH₂)₃-O, (CH₂)₄-O, CH₂CH(CH₃)-O, CH(CH₃)-CH₂-O- und CH₂CH(n-C₃H₇)-O. Bevorzugtes Beispiel von AO ist CH₂-CH₂-O (EO).

In einer Ausführungsform der vorliegenden Erfindung wird (AO)ₖ gewählt aus (CH₂CH₂O)ₖ₁, wobei k1 gewählt wird aus Zahlen im Bereich von1 bis 50.

In einer Ausführungsform der vorliegenden Erfindung wird (AO)ₖ gewählt aus -(CH₂CH₂O)ₖ₂-(CH₂CH(CH₃)-O)ₖ₃ und -(CH₂CH₂O)ₖ₂-(CH(CH₃)CH₂-O)ₓ₃, wobei k2 und k3 gleich oder verschieden sein können und aus Zahlen im Bereich von 1 bis 30 gewählt werden.

In einer Ausführungsform der vorliegenden Erfindung wird (AO)ₖ gewählt aus -(CH₂CH₂O)ₖ₄, wobei k4 im Bereich von 10 bis 50 ist, AO ist EO, und R⁸ und R⁹ unabhängig voneinander aus C₈-C₁₄-Alkyl gewählt werden.

Im Zusammenhang mit der vorliegenden Erfindung werden unter k bzw. k1, k2, k3 und k4 jeweils Mittelwerte verstanden, wobei das Zahlenmittel bevorzugt wird. Daher kann jede der Variablen k bzw. k1, k2, k3 oder k4 - so vorhanden - einen Bruch bedeuten. Ein bestimmtes Molekül kann natürlich immer nur eine ganze Zahl an AO-Einheiten tragen.

Weitere Beispiele für geeignete nicht-ionische Tenside sind Verbindungen der allgemeinen Formel (VII) und insbesondere der Formel (VII a) 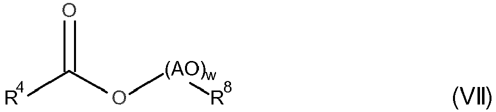 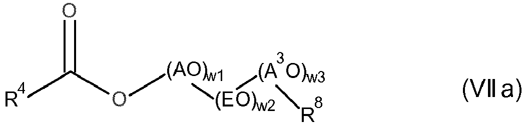 wobei
R⁴ und AO wie vorstehend definiert sind und EO Ethylenoxid, also CH₂CH₂O, bedeutet, wobei die AO in Formel (VII) und (VII a) jeweils gleich oder verschieden sein können,
R⁸ gewählt aus C₈-C₁₈-Alkyl, linear oder verzweigt
A³O gewählt wird aus Propylenoxid und Butylenoxid,
w eine Zahl im Bereich von 15 bis 70, bevorzugt 30 bis 50 ist,
w1 und w3 Zahlen im Bereich von 1 bis 5 sind und
w2 eine Zahl im Bereich von 13 bis 35 ist.

Weitere geeignete nicht-ionische Tenside sind gewählt aus Di- und Multiblockcopolymeren, aufgebaut aus Ethylenoxid und Propylenoxid. Weitere geeignete nicht-ionische Tenside sind gewählt aus ethoxylierten oder propoxylierten Sorbitanestern. Ebenfalls eignen sich Aminoxide oder Alkylglycoside. Eine Übersicht geeigneter weiterer nichtionischer Tenside findet man in EP-A 0 851 023 und in DE-A 198 19 187.

Es können auch Gemische mehrerer verschiedener nicht-ionischer Tenside enthalten sein.

Beispiele für anionische Tenside sind C₈-C₂₀-Alkylsulfate, C₈-C₂₀-Alkylsulfonate und C₈-C₂₀-Alkylethersulfate mit einer bis 6 Ethylenoxideinheiten pro Molekül.

In einer Ausführungsform der vorliegenden Erfindung kann erfindungsgemäße Formulierung im Bereich von 3 bis 20 Gew.-% Tensid enthalten.

Erfindungsgemäße Formulierungen können ein oder mehrere Enzyme enthalten. Beispiele für Enzyme sind Lipasen, Hydrolasen, Amylasen, Proteasen, Cellulasen, Esterasen, Pectinasen, Lactasen und Peroxidasen.

Erfindungsgemäße Formulierungen können beispielsweise bis 5 Gew.-% Enzym enthalten, bevorzugt sind 0,1 bis 3 Gew.-%, jeweils bezogen auf gesamten Feststoffgehalt der erfindungsgemäßen Formulierung.

Erfindungsgemäße Formulierungen können einen oder mehrere Enzymstabilisatoren enthalten. Enzymstabilisatoren dienen dem Schutz von Enzym - besonders während der Lagerung - gegen Schädigungen wie beispielsweise Inaktivierung, Denaturierung oder Zerfall etwa durch physikalische Einflüsse, Oxidation oder proteolytische Spaltung.

Beispiele von Enzymstabilisatoren sind reversible Proteaseinhibitoren, beispielsweise Benzamidin-Hydrochlorid, Borax, Borsäuren, Boronsäuren oder deren Salze oder Ester, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der vorstehend genannten Verbindungen. Auch Peptidaldehyde, das heißt Oligopeptide mit reduziertem C-Terminus, insbesondere solche aus 2 bis 50 Monomeren, werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Beispiele für Enzymstabilisatoren sind Aminoalkohole wie Mono-, Di-, Triethanol- und - Propanolamin und deren Mischungen, aliphatische Mono- und Dicarbonsäuren bis zu C₁₂-Carbonsäuren, wie beispielsweise Bernsteinsäure. Auch endgruppenverschlossene Fettsäureamidalkoxylate sind geeignete Enzymstabilisatoren.

Andere Beispiele für Enzymstabilisatoren sind Natrium-Sulfit, reduzierende Zucker und Kaliumsulfat. Ein weiteres Beispiel eines geeigneten Enzymstabilisators ist Sorbitol.

Erfindungsgemäße Formulierungen können einen oder mehrere Builder (D), insbesondere Phosphat-freie Builder (D), enthalten. Im Rahmen der vorliegenden Erfindung zählt Verbindung (A) nicht als Builder (D). Beispiele für geeignete Builder (D) sind Silikate, insbesondere Natriumdisilikat und Natriummetasilikat, Zeolithe, Schichtsilikate, insbesondere solche der Formel α-Na₂Si₂O₅ β-Na₂Si₂O₅, und δ-Na₂Si₂O₅, weiterhin Zitronensäure und ihre Natriumsalze, Bernsteinsäure und ihre Alkalimetallsalze, Fettsäuresulfonate, α-Hydroxypropionsäure, Alkalimalonate, Fettsäuresulfonate, Alkyl- und Alkenyldisuccinate, Nitrilotriessigsäure, Ethylendiamintetraessigsäure, Diethylentriaminpentaessigsäure, Hydroxyethylethylendiamintriessigsäure , Iminodibernsteinsäure, Hydroxy-iminodibernsteinsäure, Ethylendiamindibernsteinsäure, Asparaginsäurediessigsäure sowie deren Salze, weiterhin Carboxymethylinulin, Weinsäurediacetat, Weinsäuremonoacetat, oxidierte Stärke, und polymere Builder (D), beispielsweise Polycarboxylate und Polyasparaginsäure.

Ganz besonders bevorzugt enthalten erfindungsgemäße Formulierungen ein Salz der Zitronensäure, insbesondere Natriumcitrat, auch Natriumcitrat (D) genannt. Darunter wird im Zusammenhang mit der vorliegenden Erfindung vorzugsweise das Dihydrat des Trinatriumsalzes der Zitronensäure verstanden.

Bevorzugt setzt man Verbindung (A) zu Natriumcitrat (D) in einem Gewichtsverhältnis im Bereich von 10: 1 bis 1:10 ein, besonders bevorzugt beträgt das Verhältnis 3:1 bis 1:8.

Erfindungsgemäße Formulierungen können beispielsweise im Bereich von insgesamt 5 bis 40 Gew.-%, bevorzugt bis 35 Gew.-% weiteren Builder, insbesondere Natriumcitrat, enthalten, bezogen auf gesamten Feststoffgehalt der betreffenden erfindungsgemäßen Formulierung.

Ganz besonders bevorzugt enthalten erfindungsgemäße Formulierungen einen oder mehrere polymere Builder (D). Unter polymeren Buildern (D) werden dabei organische Polymere verstanden, insbesondere Polycarboxylate und Polyasparaginsäure. Polymere Builder (D) haben keine oder nur eine vernachlässigbare Wirkung als Tensid.

In einer Ausführungsform der vorliegenden Erfindung wählt man polymeren Builder (D) aus Polycarboxylaten, beispielsweise Alkalimetallsalze von (Meth)acrylsäurehomo- oder (Meth)acrylsäurecopolymeren.

Als Comonomere eignen sich monoethylenisch ungesättigte Dicarbonsäuren wie Maleinsäure, Fumarsäure, Maleinsäureanhydrid, Itaconsäure und Citraconsäure. Ein geeignetes Polymer ist insbesondere Polyacrylsäure, die bevorzugt ein mittleres Molekulargewicht M_{w} im Bereich von 2000 bis 40.000 g/mol aufweist, bevorzugt 2.000 bis 10.000 g/mol, insbesondere 3.000 bis 8.000 g/mol. Geeignet sind weiterhin copolymere Polycarboxylate, insbesondere solche der Acrylsäure mit Methacrylsäure und der Acrylsäure oder Methacrylsäure mit Maleinsäure und/oder Fumarsäure.

In einer Ausführungsform der vorliegenden Erfindung wählt man polymeren Builder (D) aus einem oder mehreren Copolymeren, hergestellt aus mindestens einem Monomeren aus der Gruppe bestehend aus monoethylenisch ungesättigten C₃-C₁₀-Mono- oder Dicarbonsäuren oder deren Anhydriden, wie Maleinsäure, Maleinsäureanhydrid, Acrylsäure, Methacrylsäure, Fumarsäure, Itaconsäure und Citraconsäure sowie mindestens einem hydrophilen oder hydrophoben Comonomeren, wie nachfolgend aufgezählt.

Geeignete hydrophobe Monomere sind beispielsweise Isobuten, Diisobuten, Buten, Penten, Hexen und Styrol, Olefine mit 10 oder mehr Kohlenstoffatomen oder deren Gemischen wie beispielsweise 1-Decen, 1-Dodecen, 1-Tetradecen, 1-Hexadecen, 1-Octadecen, 1-Eicosen, 1-Docosen, 1-Tetracosen und 1-Hexacosen, C₂₂-α-Olefin, ein Gemisch aus C₂₀-C₂₄-α-Olefinen und Polyisobuten mit im Mittel 12 bis 100 C-Atomen.

Geeignete hydrophile Monomere sind Monomere mit Sulfonat- oder Phosphonatgruppen sowie nichtionische Monomere mit Hydroxyfunktion oder Alkylenoxidguppen. Beispielsweise seien genannt: Allylalkohol, Isoprenol, Methoxypolyethylenglykol(meth)acrylat, Methoxypolypropylenglykol(meth)acrylat, Methoxypolybutylenglykol(meth)acrylat, Methoxypoly(propylenoxid-coethylenoxid)(meth)acrylat, Ethoxypolyethylenglykol(meth)acrylat, Ethoxypolypropylenglykol(meth)acrylat, Ethoxypolybutylenglykol(meth)acrylat und Ethoxypoly(propylenoxid-coethylenoxid)(meth)acrylat. Die Polyalkylenglykole enthalten dabei 3 bis 50, insbesondere 5 bis 40 und vor allem 10 bis 30 Alkylenoxideinheiten.

Besonders bevorzugte Sulfonsäuregruppen-haltige Monomere sind dabei 1-Acryl-amido-1-propansulfonsäure, 2-Acrylamido-2-propansulfonsäure, 2-Acrylamido-2-methylpropansulfonsäure, 2-Methacrylamido-2-methylpropansulfonsäure, 3-Meth-acrylamido-2-hydroxypropansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 2-Sulfoethylmethacrylat, 3-Sulfopropylmethacrylat, Sulfomethacrylamid, Sulfomethylmethacrylamid sowie Salze der vorstehend genannten Säuren, z. B. deren Natrium-, Kalium oder Ammoniumsalze.

Besonders bevorzugte Phosphonatgruppen-haltige Monomere sind die Vinylphosphonsäure und ihre Salze.

Darüber hinaus können ein oder mehrere von Pfropfpolymer (B) verschiedene amphotere Polymere als polymere Builder (D) eingesetzt werden. Beispiele für amphotere Polymere sind Copolymere von mindestens einer ethylenisch ungesättigten Carbonsäure, gewählt aus Acrylsäure und Methacrylsäure, mindestens einem Amid, gewählt aus N-C₁-C₁₀-Alkyl(meth)acrylamid, Acrylamid und Methacrylamid, und mindestens einem Comonomer, gewählt aus DADMAC, MAP-TAC und APTAC.

Erfindungsgemäße Formulierungen können beispielsweise im Bereich von insgesamt 10 bis 75 Gew.-%, bevorzugt bis 50 Gew.-% Builder (D) enthalten, bezogen auf den Feststoffgehalt der betreffenden erfindungsgemäßen Formulierung.

Erfindungsgemäße Formulierungen können beispielsweise im Bereich von insgesamt 2 bis15 Gew.-%, bevorzugt bis 10 Gew.-% polymeren Builder (D) enthalten, bezogen auf den Feststoffgehalt der betreffenden erfindungsgemäßen Formulierung.

In einer besonders bevorzugten Ausführungsform enthält erfindungsgemäße Formulierung neben Pfropfpolymer (B) einen polymerer Builder (D). Das Gewichtsverhältnis polymerer Builder (D) zu Pfropfcopolymer (B) liegt dann vorzugsweise bei 30:1 bis 3:1.

In einer Ausführungsform der vorliegenden Erfindung können erfindungsgemäße Formulierungen einen oder mehrere Cobuilder enthalten.

Beispiele für Cobuilder sind Phosphonate, beispielsweise Hydroxyalkanphosphonate und Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH-Wert 9) reagiert. Als Aminoalkanphosphonate kommen vorzugsweise Ethylendiamintetra-methylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz des DTPMP, eingesetzt.

Erfindungsgemäße Formulierungen können einen oder mehrere Alkaliträger enthalten. Alkaliträger sorgen beispielsweise für den pH-Wert von mindestens 9, wenn ein alkalischer pH-Wert gewünscht wird. Geeignet sind beispielsweise Alkalimetallcarbonate, Alkalimetallhydrogencarbonate, Alkalimetallhydroxide und Alkalimetallmetasilikate. Bevorzugtes Alkalimetall ist jeweils Kalium, besonders bevorzugt ist Natrium.

Erfindungsgemäße Formulierungen können einen oder mehrere Bleichkatalysatoren enthalten. Bleichkatalysatoren kann man wählen aus bleichverstärkenden Übergangsmetallsalzen bzw. Übergangsmetallkomplexen wie beispielsweise Mangan-, Eisen-, Cobalt-, Ruthenium- oder Molybdän-Salenkomplexe oder Mangan-, Eisen-, Cobalt-, Ruthenium- oder Molybdäncarbonylkomplexe. Auch Mangan-, Eisen-, Kobalt-, Ruthenium-, Molybdän-, Titan-, Vanadium- und Kupfer-Komplexe mit stickstoffhaltigen Tripod-Liganden sowie Kobalt-, Eisen-, Kupfer- und Ruthenium-Aminkomplexe sind als Bleichkatalysatoren verwendbar.

Erfindungsgemäße Formulierungen können einen oder mehrere Bleichaktivatoren, beispielsweise N-Methylmorpholinium-Acetonitril-Salze ("MMA-Salze"), TrimethylammoniumacetonitrilSalze, N-Acylimide wie beispielsweise N-Nonanoylsuccinimid, 1,5-Diacetyl-2,2-dioxohexahydro-1,3,5-triazin ("DADHT") oder Nitrilquats (Trimethylammoniumacetonitrilsalze) enthalten.

Weitere Beispiele für geeignete Bleichaktivatoren sind Tetraacetylethylendiamin (TAED) und Tetraacetylhexylendiamin.

Erfindungsgemäße Formulierungen können einen oder mehrere Korrosionsinhibitoren enthalten. Darunter sind im vorliegenden Fall solche Verbindungen zu verstehen, die die Korrosion von Metall inhibieren. Beispiele für geeignete Korrosionsinhibitoren sind Triazole, insbesondere Benzotriazole, Bisbenzotriazole, Aminotriazole, Alkylaminotriazole, weiterhin Phenolderivate wie beispielsweise Hydrochinon, Brenzcatechin, Hydroxyhydrochinon, Gallussäure, Phloroglucin oder Pyrogallol, weiterhin Polyethylenimin und Salze von Wismut oder Zink.

In einer Ausführungsform der vorliegenden Erfindung enthalten erfindungsgemäße Formulierungen insgesamt im Bereich von 0,1 bis 1,5 Gew.-% Korrosionsinhibitor, bezogen auf den Feststoffgehalt der betreffenden erfindungsgemäßen Formulierung.

Erfindungsgemäße Formulierungen können einen oder mehrere Gerüststoffe enthalten, beispielsweise Natriumsulfat.

Erfindungsgemäße Formulierungen können einen oder mehrere Entschäumer enthalten, gewählt beispielsweise aus Silikonölen und Paraffinölen.

In einer Ausführungsform der vorliegenden Erfindung enthalten erfindungsgemäße Formulierungen insgesamt im Bereich von 0,05 bis 0,5 Gew.-% Entschäumer, bezogen auf den Feststoffgehalt der betreffenden erfindungsgemäßen Formulierung.

In einer Ausführungsform der vorliegenden Erfindung können erfindungsgemäße Formulierungen eine oder mehrere Säuren enthalten, beispielsweise Methansulfonsäure.

In einer Ausführungsform enthalten erfindungsgemäße Formulierungen ein oder mehrere Disintegrationsmittel, auch Tablettensprengmittel genannt. Beispiele sind Stärke, Polysaccharide, beispielsweise Dextrane, weiterhin vernetztes Polyvinylpyrrolidon und Polyethylenglykolsorbitanfettsäureester.

In einer Ausführungsform der vorliegenden Erfindung enthalten solche erfindungsgemäße Formulierungen, die bei Zimmertemperatur flüssig sind, einen oder mehrere Verdicker.

Um die gewünschte Viskosität der betreffenden erfindungsgemäßen Formulierung zu erreichen, setzt man gelförmigen erfindungsgemäßen Formulierungen vorzugsweise ein oder mehrere Verdickungsmittel zu, wobei es sich als besonders vorteilhaft erweist, wenn die betreffende erfindungsgemäße Formulierung Verdickungsmittel im Bereich von 0, 1 bis 8 Gew. -%, bevorzugt von 0,2 bis 6 Gew.-% und besonders bevorzugt von 0,2 bis 4 Gew. -% enthält, bezogen auf den Feststoffgehalt der betreffenden erfindungsgemäßen Formulierung.

Als Verdickungsmittel kann man aus der Natur stammende Polymere oder abgewandelte Naturstoffe oder synthetische Verdickungsmittel wählen.

Als Beispiele für aus der Natur stammende Polymere, die als Verdickungsmittel im Rahmen der vorliegenden Erfindung geeignet sind, sind zu nennen: Agar-Agar, Carrageen, Tragant, Gummi arabicum, Alginate, Pektine, Polyosen, Guar-Mehl, Johannisbrotbaumkernmehl, Stärke, Dextrine, Xanthan, Gelatine und Casein.

Beispiele für Verdickungsmittel aus der Gruppe der abgewandelten Naturstoffe kann man beispielsweise aus der Gruppe der modifizierten Stärken und Cellulosen wählen. Beispielhaft seien Carboxymethylcellulose und andere Celluloseether, Hydroxyethylcellulose- und Hydroxypropy-lcellulose sowie Kernmehlether genannt.

Synthetische Verdickungsmittel sind gewählt aus teilweise vernetzten Poly(meth)acrylsäuren, hydrophob modifizierten Polyurethanen (HEUR Verdicker) und mit Fettalkoholethoxylaten veresterten Poly(meth)acrylsäure-Copolymeren (HASE Verdicker).

Ein besonders bevorzugt verwendetes Verdickungsmittel ist Xanthan.

In einer Ausführungsform der vorliegenden Erfindung können erfindungsgemäße Formulierungen ein oder mehrere organische Lösungsmittel enthalten. Beispielsweise kann man organische Lösungsmittel aus den Gruppen der Mono-Alkohole, Diole, Triole bzw. Polyole, der Ether, Ester und/oder Amide wählen. Besonders bevorzugt sind dabei organische Lösungsmittel, die wasserlöslich sind, wobei "wasserlösliche" Lösungsmittel im Sinne der vorliegenden Anmeldung Lösungsmittel sind, die bei Zimmertemperatur mit Wasser vollständig, d. h. ohne Mischungslücke, mischbar sind.

Organische Lösungsmittel, die für erfindungsgemäße Formulierungen geeignet sind, wählt man vorzugsweise aus der Gruppe ein- oder mehrwertigen Alkohole, Alkanolamine oder Glykolether, die im angegebenen Konzentrationsbereich mit Wasser mischbar sind. Vorzugsweise wählt man organische Lösungsmittel aus Ethanol, n- oder i-Propanol, Butanolen, Glykol, 1,2-Propandiol, oder Butandiol, Glycerin, Diglykol, Propyl- oder n-Butyldiglykol, Hexylenglycol, Ethylenglykolmethylether, Ethylenglykolethylether, Ethylenglykolpropylether, Ethylenglykolmono-n-butylether, Diethylenglykolmethylether, Di-ethylenglykolethylether, Propylenglykolmethyl-, - ethyl- oder -propylether, Dipropylenglykolmethyl-, oder -ethylether, Methoxy-, Ethoxy oder Butoxytriglykol, 1-Butoxyethoxy-2-propanol, 3-Methyl-3-methoxybutanol, Propylenglykol-t-butylether sowie Mischungen von zwei oder mehr der vorstehend genannten organischen Lösungsmittel.

In einer Ausführungsform der vorliegenden Erfindung weisen erfindungsgemäße Formulierungen einen pH-Wert im Bereich von 6 bis 14 auf, bevorzugt 8 bis 13. Dabei wird im Falle von solchen erfindungsgemäßen Formulierungen, die bei Zimmertemperatur fest sind, der pH-Wert einer 1 Gew.-% wässrigen Lösung oder der flüssigen Phase einer 1 Gew.-% wässrigen Suspension bestimmt.

Erfindungsgemäße Formulierungen eignen sich sehr gut als oder zur Herstellung von Geschirrspülmitteln, insbesondere für die maschinelle Geschirrreinigung (englisch "Automatic Dishwashing" oder kurz ADW). Erfindungsgemäße Formulierungen selbst und aus erfindungsgemäßen Formulierungen hergestellte Geschirrspülmittel - insbesondere aus erfindungsgemäßen Formulierungen hergestellte Phosphat-freie Geschirrspülmittel -weisen beim Geschirrspülen eine sehr gute Belagsinhibierung insbesondere auf Spülgut aus Glas auf. Insbesondere sind erfindungsgemäße Formulierungen auch gegen hartnäckige Flecken wirksam, beispielsweise gegen Teeflecken und Teereste.

Beispiele für Spülgut aus Metall sind Bestecke, Töpfe, Pfannen und Knoblauchpressen, insbesondere Besteckteile wie Messer, Tortenheber und Vorlagebestecke.

Als Beispiele für Spülgut aus Glas seien dabei genannt: Gläser, gläserne Schüsseln, gläsernes Geschirr wie beispielsweise Glasteller, aber auch Gegenstände, die mindestens eine Oberfläche aus Glas aufweisen, das dekoriert oder nicht dekoriert sein kann, beispielsweise gläserne Vasen, durchsichtige Topfdeckel und Glasgefäße zum Kochen.

Als Beispiele für Spülgut aus Kunststoff seien dabei Teller, Tassen, Becher und Schüsseln aus Melamin, Polystyrol und Polyethylen genannt.

Als Beispiele für Spülgut aus Porzellan seien dabei Teller, Tassen, Becher und Schüsseln aus Porzellan, weiß oder farbig, jeweils mit oder ohne Dekoration, genannt.

Ein weiterer Gegenstand der vorliegenden Erfindung ist daher die Verwendung von erfindungsgemäßen Formulierungen zum Spülen von Geschirr und Küchenutensilien, und zwar insbesondere zur maschinellen Geschirrreinigung, also zum Spülen mit einer Spülmaschine. Ein weiterer Gegenstand der vorliegenden Erfindung ist ein Verfahren zur maschinellen Geschirrreinigung unter Verwendung von mindestens einer erfindungsgemäßen Formulierung, im Rahmen der vorliegenden Erfindung auch erfindungsgemäßes Geschirrspülverfahren genannt. Zur Durchführung des erfindungsgemäßen Geschirrspülverfahrens kann man so vorgehen, dass man Geschirr oder Küchenutensilien mit einer wässrigen Lösung oder Suspension, enthaltend mindestens eine erfindungsgemäße Formulierung, in Kontakt bringt. Nach dem Inkontaktbringen kann man einwirken lassen. Anschließend entfernt man die so erhältliche Flotte, spült ein- oder mehrfach mit vorzugsweise klarem Wasser nach und lässt trocknen.

In einer Ausführungsform der vorliegenden Erfindung setzt man zum Reinigen Wasser mit einer Härte im Bereich von 1 bis 30 °dH, bevorzugt 2 bis 25 °dH ein, wobei unter deutscher Härte insbesondere die Summe aus Magnesium-Härte und Calcium-Härte zu verstehen ist.

In einer besonderen Variante des erfindungsgemäßen Geschirrspülverfahrens setzt man weder Regeneriersalz noch separaten Klarspüler ein.

Ein weiterer Gegenstand der vorliegenden Erfindung ist ein Verfahren zur Herstellung von erfindungsgemäßen Formulierungen, im Rahmen der vorliegenden Erfindung auch erfindungsgemäßes Herstellverfahren genannt. Das erfindungsgemäße Herstellverfahren ist dadurch gekennzeichnet, dass man mindestens eine Verbindung (A), mindestens ein Pfropfcopolymer (B) und gegebenenfalls einen oder mehrere weitere Inhaltsstoffe (D) und gegebenenfalls Peroxid (C) bzw. Chlorbleichmittel (C) in einem oder mehreren Schritten miteinander vermischt und anschließend gegebenenfalls Wasser ganz oder partiell entfernt.

Verbindung (A), Pfropfcopolymer (B), Peroxid (C) und weitere Inhaltsstoffe (D) sind vorstehend beschrieben.

In einer anderen Ausführungsform der vorliegenden Erfindung vermischt man Verbindung (A), einen oder mehrere weitere Inhaltsstoffe (D) und gegebenenfalls Peroxid (C) in trockener Form und gibt dann eine wässrige Lösung von Pfropfcopolymer (B) zu, entweder außerhalb oder innerhalb einer Spülmaschine.

In einer anderen Ausführungsform der vorliegenden Erfindung vermischt man Verbindung (A), Pfropfcopolymer (B) und einen oder mehrere weitere Inhaltsstoffe (D) und gegebenenfalls Peroxid (C) bzw. Chlorbleichmittel (C) in trockener Form und verpresst die so erhaltene Mischung zu Formköpern, insbesondere zu Tabletten.

In einer Ausführungsform der vorliegenden Erfindung kann man, bevor man das Wasser zumindest teilweise entfernt, mit einem oder mehreren weiteren Inhaltsstoffen (D) für erfindungsgemäße Formulierung vermischen, beispielsweise mit einem oder mehreren Tensiden, einem oder mehreren Enzymen, einem oder mehreren Enzymstabilisatoren, einem oder mehreren Buildern (D), bevorzugt einem oder mehreren Phosphat-freien Buildern (D), insbesondere einem oder mehreren Polymeren Buildern (D), einem oder mehrere Cobuilder, einem oder mehreren Alkaliträgern, einem oder mehreren Bleichkatalysatoren, einem oder mehreren Bleichaktivatoren, einem oder mehreren Bleichmittelstabilisatoren, einem oder mehreren Entschäumern, einem oder mehreren Korrosionsinhibitoren, einem oder mehreren Gerüststoffen, mit Puffer oder Farbstoff.

In einer Ausführungsform geht man so vor, dass man das Wasser ganz oder teilweise, beispielsweise bis zu einer Restfeuchte im Bereich von null bis 15 Gew.-%, bevorzugt 0,1 bis 10 Gew.-% aus erfindungsgemäßer Formulierung entfernt, indem man es verdampft, insbesondere durch Sprühtrocknung, Sprühgranulierung oder Kompaktierung.

In einer Ausführungsform der vorliegenden Erfindung entfernt man das Wasser, ganz oder teilweise, bei einem Druck im Bereich von 0,3 bis 2 bar.

In einer Ausführungsform der vorliegenden Erfindung entfernt man das Wasser, ganz oder teilweise, bei Temperaturen im Bereich von 60 bis 220°C.

In einer anderen Ausführungsform entfernt man das Wasser nicht. Stattdessen kann man weiteres Wasser hinzufügen. Besonders bevorzugt gibt man außerdem einen Verdickungsmittel zu. Auf diesem Weg kann man erfindungsgemäße flüssige Formulierungen erhalten. Bei Zimmertemperatur können flüssige erfindungsgemäße Formulierungen beispielsweise in Gelform vorliegen.

Durch das erfindungsgemäße Herstellungsverfahren kann man erfindungsgemäße Formulierungen leicht erhalten.

Die erfindungsgemäßen Formulierungen können flüssig oder fest, ein- oder mehrphasig, als Tabletten oder in Form anderer Dosiereinheiten, beispielsweise als sogenannte Pouches, verpackt oder unverpackt bereitgestellt werden.

Ein weiterer Gegenstand der vorliegenden Erfindung sind Pfropfcopolymere, im Rahmen der vorliegenden Erfindung auch kurz Pfropfcopolymer (B) oder erfindungsgemäßes Pfropfcopolymer genannt. Erfindungsgemäße Pfropfcopolymere sind aufgebaut aus
(a) mindestens einer Pfropfgrundlage, gewählt aus nicht-ionischen Monosacchariden, Disacchariden, Oligosacchariden und Polysacchariden,
   und Seitenketten, erhältlich durch Aufpfropfen von
(b) mindestens einer ethylenisch ungesättigten Mono- oder Dicarbonsäure und
(c) mindestens einer Verbindung der allgemeinen Formel (I), 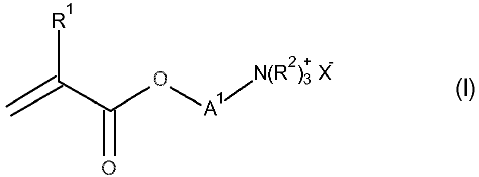
wobei die Variablen wie folgt definiert sind:
R¹ ist gewählt aus Methyl und Wasserstoff,
A¹ ist gewählt aus C₂-C₄-Alkylen,
R² sind gleich oder verschieden und gewählt aus C₁-C₄-Alkyl,
X- ist gewählt aus Halogenid, Mono-C₁-C₄-Alkylsulfat und Sulfat.

Beispiele für Mono-C₁-C₄-Alkylsulfat sind Methylsulfat, Ethylsulfat, iso-Propylsulfat und n-Butylsulfat, bevorzugt sind Methylsulfat und Ethylsulfat. Wenn man X⁻ als Sulfat wählt, so steht X⁻ für ein halbes Äquivalent Sulfat.

Dabei sind die Variablen wie folgt definiert:
R¹ ist gewählt aus Methyl und Wasserstoff,
A¹ ist gewählt aus C₂-C₄-Alkylen, beispielsweise -CH₂-CH₂-, CH₂-CH(CH₃)-, -(CH₂)₃-, -(CH₂)₄-, bevorzugt sind -CH₂-CH₂- und -(CH₂)₃-,
R² sind verschieden oder vorzugsweise gleich und gewählt aus C₁-C₄-Alkyl, beispielsweise Methyl, Ethyl, n-Propyl, n-Butyl, iso-Propyl, iso-Butyl, sec.-Butyl, tert.-Butyl, bevorzugt sind mindestens zwei R² gleich und jeweils Methyl, und die dritte Gruppe R² ist Ethyl, n-Propyl oder n-Butyl, oder zwei R² sind gleich und jeweils Ethyl, und die dritte Gruppe R² ist Methyl, n-Propyl oder n-Butyl. Besonders bevorzugt sind alle drei R² jeweils gleich und gewählt aus Methyl.
X⁻ ist gewählt aus Halogenid, beispielsweise lodid, Bromid und insbesondere Chlorid, weiterhin aus Mono-C₁-C₄-Alkylsulfat und Sulfat.

In einer bevorzugten Ausführungsform der vorliegenden Erfindung sind in Monomer (c) die Variablen wie folgt gewählt:
R¹ ist Wasserstoff oder Methyl,
R² sind gleich und jeweils Methyl,
A¹ ist CH₂CH₂, und
X- ist Chlorid.

In einer Ausführungsform der vorliegenden Erfindung ist Monomer (c) gewählt aus

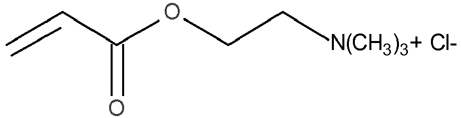

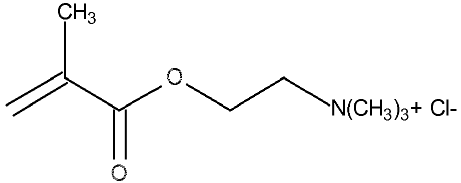

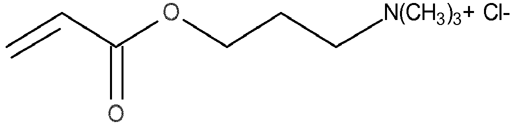

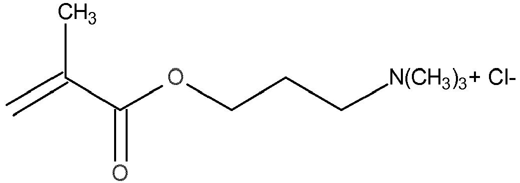

Erfindungsgemäßes Pfropfcopolymer (B) kann in einer oder mehreren Seitenketten mindestens ein weiteres Comonomer (d) einpolymerisiert enthalten, beispielsweise Hydroxyalkylester wie 2-Hydroxyethyl-(meth)acrylat oder 3-Hydroxypropyl(meth)acrylat, oder Ester von alkoxylierten Fettalkoholen, oder Sulfonsäuregruppen-haltige Comonomere, beispielsweise 2-Acrylamido-2-methylpropansulfonsäure (AMPS) und ihre Alkalimetallsalze.

Vorzugsweise enthält erfindungsgemäßes Pfropfcopolymer (B) außer Monomer (c) und Monocarbonsäure (b) bzw. Dicarbonsäure (b) keine weiteren Comonomere (d) in einer oder mehreren Seitenketten.

In einer Ausführungsform der vorliegenden Erfindung ist der Anteil von Pfropfgrundlage (a) in erfindungsgemäßem Pfropfcopolymer (B) im Bereich von 40 bis 95 Gew.-%, vorzugsweise von 50 bis 90 Gew.-%, jeweils bezogen auf gesamtes erfindungsgemäßes Pfropfcopolymer (B).

In einer Ausführungsform der vorliegenden Erfindung ist der Anteil von Monocarbonsäure (b) bzw. Dicarbonsäure (b) im Bereich von 2 bis 40 Gew.-%, vorzugsweise von 5 bis 30 Gew.-% und insbesondere von 5 bis 25 Gew.-%, jeweils bezogen auf gesamtes erfindungsgemäßes Pfropfcopolymer (B).

Die Monomeren vom Typ (c) sind in Mengen von 5 bis 50 Gew.-%, vorzugsweise von 5 bis 40 Gew.-% und besonders bevorzugt von 5 bis 30 Gew.-% einpolymerisiert, jeweils bezogen auf gesamtes erfindungsgemäßes Pfropfcopolymer (B).

Es ist bevorzugt, wenn erfindungsgemäßes Pfropfcopolymer mehr Monocarbonsäure (b) als Verbindung (c) einpolymerisiert enthält, und zwar auf die molaren Anteile bezogen, beispielsweise im Bereich von 1,1:1 bis 5:1, bevorzugt 2:1 bis 4:1.

In einer Ausführungsform der vorliegenden Erfindung ist das mittlere Molekulargewicht (M_{w}) von Pfropfcopolymer (B) im Bereich von 2.000 bis 200.000 g/mol, vorzugsweise von 5.000 bis 150.000 und insbesondere im Bereich von 8.000 bis 100.000 g/mol. Das mittlere Molekulargewicht M_{w} misst man vorzugsweise durch Gelpermeationschromatographie in wässriger KCI/Ameisensäure-Lösung.

Ein weiterer Gegenstand der vorliegenden Erfindung ist ein Verfahren zur Herstellung von erfindungsgemäßen Pfropfcopolymeren, im Rahmen der vorliegenden Erfindung auch kurz erfindungsgemäßes Verfahren genannt. Zur Durchführung des erfindungsgemäßen Verfahrens kann man so vorgehen, dass man
(b) mindestens eine ethylenisch ungesättigte Mono- oder Dicarbonsäure und (c) mindestens eine Verbindung der allgemeinen Formel (I) in Gegenwart von mindestens einer Pfropfgrundlage (a)
radikalisch copolymerisiert.

Monocarbonsäure (b), Dicarbonsäure (b), Pfropfgrundlage (a) und Monomer (c) sind vorstehend beschrieben.

Man führt das erfindungsgemäße Verfahren bevorzugt in Wasser als Lösungsmittel durch. Gegebenenfalls kann man statt Wasser ein Gemische aus Wasser und einem oder mehreren organischen Lösemitteln wie beispielsweise Alkohole und Ketone, aber auch dipolar-aprotische, mit Wasser mischbaren Lösemitteln wie z.B. DMSO, DMF oder NMP einsetzen.

In einer Ausführungsform der vorliegenden Erfindung führt man das erfindungsgemäße Verfahren bei einer Temperatur im Bereich von 60 bis 120°C, bevorzugt 65 bis 100°C, ganz besonders bevorzugt bei 70 bis 90°C durch.

In einer Ausführungsform der vorliegenden Erfindung führt man das erfindungsgemäße Verfahren bei Normaldruck durch. In einer anderen Ausführungsform führt man das erfindungsgemäße Verfahren bei einem Druck im Bereich von 1,2 bis 20 bar durch.

In einer bevorzugten Variante legt man Pfropfgrundlage (a) in wässriger Lösung vor und gibt dann Monomer (c) und Monocarbonsäure (b) oder Dicarbonsäure (b) in Gegenwart von Radikalstarter zu.

Das erfindungsgemäße Verfahren kann man bevorzugt so durchführen, dass man Monomer (c) und Monocarbonsäure (b) bzw. Dicarbonsäure (b) nicht vollständig mit Pfropfgrundlage (a) zur Reaktion bringt, sondern z.B. durch portionsweise oder kontinuierliche Zugabe mit Pfropfgrundlage (a) reagieren lässt.

In einer anderen Variante geht man so vor, dass man zunächst nur einen Teil an Monomer (c) und Monocarbonsäure (b) bzw. Dicarbonsäure (b) sowie Radikalstarter zur Pfropfgrundlage (a) gibt, den Rest in Mischung mit Monomer (c) und Monocarbonsäure (b) bzw. Dicarbonsäure (b) nebeneinander, wobei bei jeder Zugabe von Monomer (c) und Monocarbonsäure (b) bzw. Dicarbonsäure (b) auch Radikalstarter zugesetzt wird.

In einer besonders bevorzugten Variante legt man zunächst eine wässrige Lösung von Pfropfgrundlage (a) vor und erwärmt auf 60 bis 120°C. Anschließend gibt man, vorzugsweise kontinuierlich, eine Teilmenge von Monomeren (c) zusammen mit einem Radikalstarter zu. Nach dem Abklingen der Reaktion mit der Pfropfgrundlage (a) setzt man, vorzugsweise kontinuierlich, eine Mischung von Monocarbonsäure (b) bzw. Dicarbonsäure (b) und der restlichen Menge an Monomer (c) zusammen mit weiterem Radikalstarter zu.

Als Radikalstarter sind beispielsweise geeignet: Azodiisobutyronitril (AIBN), Peroxide wie z.B. Benzoylperoxid, ferner Hydroperoxide und Perester. Besonders bevorzugt ist die Verwendung von Natriumperoxodisulfat und tert.-Butylhydroperoxid bzw. von Wasserstoffperoxid, die man in den handelsüblichen Konzentrationen und Zubereitungen, z.B. als wässerige oder alkoholische Lösungen verwenden kann. In einer anderen Ausführungsform kann man eine Mischung von H₂O₂ mit Eisen(II)salzen zum Einsatz kommen. Das Wasserstoffperoxid wird dabei vorzugsweise in Form wässeriger Lösungen verwendet. Radikalstarter setzt man vorzugsweise in Mengen von 0,001 bis 30 Mol-%, vorzugsweise von 0,1 bis 25 Mol-% und insbesondere von 1 bis 20 Mol-%, jeweils bezogen auf die Summe an molaren Mengen an Monomer (c) und Monocarbonsäure (b) bzw. Dicarbonsäure (b) ein.

Man kann Monomer (c) als solches in Pfropfcopolymer (B) einpolymerisieren oder ein nicht quaternisertes Äquivalent, beispielsweise im Falle des Halogenids oder Sulfats oder C₁-C₄-Alkylsulfats von Trimethylammoniumethyl(meth)acrylat durch ersatzweises Einpolymerisieren von 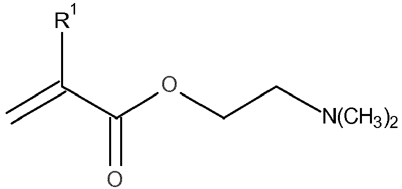 und im Falle des Halogenids oder Sulfats oder C₁-C₄-Alkylsulfats von Trimethylammoniumpropyl(meth)acrylat durch ersatzweises Einpolymerisieren von

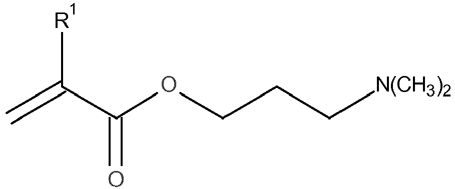

Im Anschluss an die Copolymerisation alkyliert man, beispielsweise mit C₁-C₄-Alkylhalogenid oder C₁-C₄-Dialkylsulfat, beispielsweise mit Ethylchlorid, Ethylbromid, Methylchlorid, Methylbromid, Dimethylsulfat oder Diethylsulfat.

In einer Ausführungsform der vorliegenden Erfindung kann man nach Beendigung der Zugabe von Monocarbonsäure (b) bzw. Dicarbonsäure (b), Monomer (c) und Radikalstarter weiteren Radikalstarter zugeben, besonders bevorzugt im kontinuierlichen Zulaufverfahren. Dadurch kann man den Gehalt an Monocarbonsäure (b) bzw. Dicarbonsäure (b) und Monomer (c) in erfindungsgemäßem Pfropfcopolymer (B) senken.

In einer Ausführungsform der vorliegenden Erfindung kann man nach beendeter Polymerisation bleichen, beispielsweise mit Peroxid wie H₂O₂.

In einer Ausführungsform der vorliegenden Erfindung kann man nach beendeter Polymerisation Restmonomer entfernen, insbesondere Monocarbonsäure (b) bzw. Dicarbonsäure (b) kann man durch Wasserdampfdestillation zumindest weitgehend entfernen.

Erfindungsgemäßes Pfropfcopolymer (B) kann man vorzugsweise als wässrige Lösung erhalten, aus der man es isolieren kann, z. B. durch Sprühtrocknung, Sprühgranulierung oder Gefriertrocknung.

Wünscht man erfindungsgemäßes Pfropfcopolymer (B) in Form einer wässrigen Lösung zu verarbeiten oder zu lagern, so ist es bevorzugt, mindestens ein Biozid zuzusetzen.

Wahlweise kann man Lösung von erfindungsgemäßem Pfropfcopolymer oder getrocknetes erfindungsgemäßes Pfropfcopolymer zur Herstellung der erfindungsgemäßen Formulierungen verwenden.

Die Erfindung wird durch Arbeitsbeispiele weiter erläutert,
Beispiele

Allgemeine Anmerkungen zu den Versuchen zur Belagsinhibierung

Alle Spülversuche wurden in einer Geschirrspülmaschine der Fa. Miele, Typ G1222 SCL durchgeführt. Dabei wurde das Programm mit 65°C für den Spülgang und 65°C für den Klarspülgang gewählt. Die Prüfungen wurden mit aufgehärtetem Wasser mit einer Wasserhärte von 21 °dH (Ca/Mg):HCO₃ (3:1):1.35 durchgeführt. Es wurde kein separater Klarspüler zugegeben und die eingebaute Wasserenthärtung (Ionentauscher) nicht mit Regeneriersalz regeneriert. Es wurde pro Spülgang jeweils 18 g der genannten erfindungsgemäßen Formulierung dosiert. Am Anfang eines jeden Spülgangs wurden 50 g eines Ballastschmutzes zugegeben, bestehend aus Fett, Protein und Stärke.

Zur Beurteilung der Belagsinhibierung wurden insgesamt 30 aufeinander folgende Spülversuche mit demselben Testspülgut durchgeführt. Als Testspülgut dienten in jedem Spülversuch drei Edelstahl-Messer, drei blaue Melaminteller, drei Trinkgläser und drei Porzellanteller. Zwischen zwei Spülversuchen wurde jeweils eine Stunde gewartet, davon 10 min mit geschlossener Tür und 50 min mit geöffneter Tür der Geschirrspülmaschine.

Nach Beendigung des 30. Spülversuchs wurde das Spülgut nach dem Trocknen aus der Maschine entfernt.

Die Gläser wurden an einem Gerät zur digitalen Bildausanalyse an gekrümmten Oberflächen mit einer Zeilenkamera fotografiert. Mittels einer Software zur Bildanalyse wurden für die Bilder unterschiedliche Werte berechnet (Weiss Imaging and Solutions GmbH, siehe z.B. SÖFW, 133, 10,2007, S. 48-52). Aussagekräftig für den Belag des Glases ist der Mittelwert des Grauwertes über die ausgewertete Fläche. Dieser umfasst bei den verwendeten Gläsern (Gesamthöhe von 13,5 cm) den Bereich ab 2 cm vom Boden und 2,5 cm vom oberen Rand.

### I. Herstellung von erfindungsgemäßen Pfropfcopolymeren (B), von erfindungsgemäßen Formulierungen und von Vergleichsformulierungen

Eingesetzte Comonomere:
(a.1): Maltodextrin, kommerziell erhältlich als Cargill C*Dry MD01955
(b.1): Acrylsäure
(c.1): [2-(Methacryloyloxy)ethyl]trimethylammoniumchlorid ("TMAEMC")

Im Rahmen der vorliegenden Anmeldung sind Angaben in % Gewichtsprozent, wenn nicht ausdrücklich anders angegeben.

Als Biozid verwendete man stets eine 9 Gew.-% Lösung von 1,2-Benzisothiazolin-3-on in Wasser-Propylenglykol-Gemisch, kommerziell erhältlich als Proxel™ XL2 Antimicrobial. Mengenangaben sind tell qu'elle.

### I.1 Herstellung von erfindungsgemäßem Pfropfcopolymer (B.1)

In einem Rührreaktor wurden 235 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 28,8 g (c.1) in 146 g Wasser, innerhalb von 4 Stunden.
b) Eine Lösung von 7,88 g Natriumperoxodisulfat in 68,0 g Wasser innerhalb von 5 h, gleichzeitig beginnend mit der Dosierung von a).
c) Eine Lösung aus 29,9 g (b.1) und 33,3 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 0,58 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 23,2 Gew.-% Lösung des erfindungsgemäßen Pfropfcopolymers (B.1).

### 1.2 Herstellung von erfindungsgemäßem Pfropfcopolymer (B.2)

In einem Rührreaktor wurden 235 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 43,6 g (c.1) in 150 g Wasser, innerhalb von 4 Stunden.
b) Eine Lösung von 7,88 g Natriumperoxodisulfat in 68,0 g Wasser innerhalb von 5 h, gleichzeitig beginnend mit der Dosierung von a).
c) Eine Lösung aus 15,1 g (b.1) und 16,8 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 0,59 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 23,2 Gew.-% Lösung des erfindungsgemäßen Pfropfcopolymers (B.2).

### 1.3 Herstellung von erfindungsgemäßem Pfropfcopolymer (B.3)

In einem Rührreaktor wurden 235 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 47,7 g (c.1) in 151 g Wasser, innerhalb von 4 Stunden.
b) Eine Lösung von 7,88 g Natriumperoxodisulfat in 68,0 g Wasser innerhalb von 5 h, gleichzeitig beginnend mit der Dosierung von a).
c) Eine Lösung aus 11,0 g (b.1) und 12,2 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 0,59 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 23,2 Gew.-% Lösung des erfindungsgemäßen Pfropfcopolymers (B.3).

### I.4 Herstellung von erfindungsgemäßem Pfropfcopolymer (B.4)

In einem Rührreaktor wurden 220 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 40,6 g (c.1) in 149 g Wasser, innerhalb von 4 Stunden.
b) Eine Lösung von 9,85 g Natriumperoxodisulfat in 68,0 g Wasser innerhalb von 5 h, gleichzeitig beginnend mit der Dosierung von a).
c) Eine Lösung aus 32,8 g (b.1) und 36,5 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 0,73 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 22,4 Gew.-% Lösung des erfindungsgemäßen Pfropfcopolymers (B.4).

### 1.5 Herstellung von erfindungsgemäßem Pfropfcopolymer (B.5)

In einem Rührreaktor wurden 176 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 87,1 g (c.1) in 161 g Wasser, innerhalb von 4 Stunden.
b) Eine Lösung von 15,8 g Natriumperoxodisulfat in 68,0 g Wasser innerhalb von 5 h, gleichzeitig beginnend mit der Dosierung von a).
c) Eine Lösung aus 30,2 g (b.1) und 33,6 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 1,16 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 22,7 Gew.-% Lösung des erfindungsgemäßen Pfropfcopolymers (B.5).

### 1.6 Herstellung von erfindungsgemäßem Pfropfcopolymer (B.6)

In einem Rührreaktor wurden 145 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 80,1 g (c.1) in 159 g Wasser, innerhalb von 4 Stunden.
b) Eine Lösung von 19,5 g Natriumperoxodisulfat in 68,0 g Wasser innerhalb von 5 h, gleichzeitig beginnend mit der Dosierung von a).
c) Eine Lösung aus 64,9 g (b.1) und 72,0 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 1,45 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 22,9 Gew.-% Lösung des erfindungsgemäßen Pfropfcopolymers (B.6).

### 1.7 Herstellung von erfindungsgemäßem Pfropfcopolymer (B.7)

In einem Rührreaktor wurden 147 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 49,5 g (c.1) in 152 g Wasser, innerhalb von 4 Stunden.
b) Eine Lösung von 19,7 g Natriumperoxodisulfat in 68,0 g Wasser innerhalb von 5 h, gleichzeitig beginnend mit der Dosierung von a).
c) Eine Lösung aus 97,3 g (b.1) und 108 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 1,46 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 24,0 Gew.-% Lösung des erfindungsgemäßen Pfropfcopolymers (B.7).

### 1.8 Herstellung von erfindungsgemäßem Pfropfcopolymer (B.8)

In einem Rührreaktor wurden 147 g (a.1) in 618 g Wasser vorgelegt und unter Rühren auf 80°C erhitzt. Bei 80°C wurden simultan und über getrennte Zuläufe folgende Lösungen folgendermaßen zudosiert:
a) Eine wässrige Lösung aus 96,5 g (c.1) in 163 g Wasser, innerhalb von 4 Stunden.
b) Eine Lösung von 19,7 g Natriumperoxodisulfat in 68,0 g Wasser innerhalb von 5 h, gleichzeitig beginnend mit der Dosierung von a).
c) Eine Lösung aus 50,2 g (b.1) und 55,8 g Natronlauge (50%ig in Wasser), verdünnt mit 139 g Wasser, innerhalb von 2 Stunden, beginnend 2 Stunden nach Dosierungsbeginn von a).

Nach vollständiger Zugabe der Lösungen a) bis c) wurde die Reaktionsmischung eine Stunde bei 80°C gerührt. Anschließend wurde eine Lösung von 1,46 g Natriumperoxodisulfat in 10,0 g Wasser zugegeben und weitere 2 Stunden bei 80°C gerührt. Anschließend kühlte man auf Zimmertemperatur ab und gab 8 g Biozid zu. Man erhielt eine 23,2 Gew.-% Lösung des erfindungsgemäßen Pfropfcopolymers (B.8).

### 1.9 Vergleichsbeispiel

Herstellung eines Vergleichs-Pfropfcopolymers (V-9)
Vergleichs-Pfropfcopolymer V-9 wurde gemäß Beispiel 4 aus EP 2 138 560 B1 hergestellt.

### Herstellung von erfindungsgemäßer Formulierung F.1 bis F.8 und von Vergleichsformulierung V-F.9

Es wurden erfindungsgemäße Formulierungen F.1 bis F.8 und Vergleichsformulierungen V-F.9 hergestellt, indem man die Komponenten gemäß Tabelle 1 - mit Ausnahme von Tensid 1 - trocken vermischte. Nichtionisches Tensid 1 schmolz man auf und rührte in die trockene Mischung ein und verteilte es dadurch möglichst homogen. Liegt Pfropfcopolymer (B.1) als wässrige Lösung vor, so kann man das Pfropfcopolymer zunächst durch Trocknen isolieren und fest zu den anderen festen Komponenten geben oder separat als Lösung in die Spülmaschine zugeben. Die Komponenten der erfindungsgemäßen Formulierungen F.1 bis F.8 und Vergleichsformulierung V-F.9 gehen aus Tabelle 1 hervor.

**Tabelle 1: Zusammensetzung von erfindungsgemäßen Formulierungen F.1 bis F.4 und Vergleichsformulierung V-F.9**

| Bestandteil [g] | F.1 | F.2 | F.3 | F.4 | V-F.9 |
|---|---|---|---|---|---|
| (A.1) | 10 | 10 | 10 | 10 | 10 |
| Trinatriumzitrat Dihydrat | 35 | 35 | 35 | 35 | 35 |
| (B) | 1 (B.1) | 1 (B.2) | 1 (B.3) | 1 (B.4) | - |
| V-9 | | | | | 1 |
| Polymerer Builder (D.1) | 9 | 9 | 9 | 9 | 9 |
| (C.1) | 10,2 | 10,2 | 10,2 | 10,2 | 10,2 |
| Nicht-ionisches Tensid 1 | 4 | 4 | 4 | 4 | 4 |
| Nicht-ionisches Tensid 2 | 1 | 1 | 1 | 1 | 1 |
| Protease | 2,5 | 2,5 | 2,5 | 2,5 | 2,5 |
| Amylase | 1 | 1 | 1 | 1 | 1 |
| Na₂Si₂O₅ | 2 | 2 | 2 | 2 | 2 |
| TAED | 4 | 4 | 4 | 4 | 4 |
| Na₂CO₃ | 19,5 | 19,5 | 19,5 | 19,5 | 19,5 |
| HEDP | 0,8 | 0,8 | 0,8 | 0,8 | 0,8 |

| | | | | | |
|---|---|---|---|---|---|
| Erläuterung: (A.1): MGDA-Na₃, 78 Gew.-%, Rest ist Wasser (C.1): Natriumpercarbonat, 2 Na₂CO₃·3 H₂O₂ Nicht-ionisches Tensid 1: n-C₈H₁₇-CH(OH)-CH₂-O-(EO)₂₂-CH(CH₃)-CH₂-O-n-C₁₀H₂₁ Nicht-ionisches Tensid 2: n-C₁₀H₂₁-CH(OH)-CH₂-O-(EO)₄₀-n-C₁₀H₂₁ Na₂Si₂O₅: kommerziell als Britesil® H 265 LC HEDP: 1-Hydroxyethan-1,1-diphosphonat Dinatriumsalz Polymerer Builder (D.1): Polyacrylsäure M_{w} 4.000 g/mol als Natriumsalz, vollständig neutralisiert | | | | | |

In erfindungsgemäßer Formulierung F.5 wurde erfindungsgemäßes Pfropfcopolymer (B.1) durch eine gleiche Menge an (B.5), also 1 g, ersetzt. Für die erfindungsgemäßen Formulierungen F.6 bis F.8 gilt mutatis mutandis.

### II. Versuche zur Belagsinhibierung

Die Grauwerte der drei Gläser pro Versuch wurden für jede Formulierung gemittelt. Je höher der Grauwert, desto stärker ist das Filming auf dem Glas.

Die Differenz der Grauwerte von Formulierung V-F.9 zur betreffenden erfindungsgemäßen Formulierung ist in Tabelle 2 dargestellt. Je negativer der Wert, desto größer der Vorteil in der Belagsinhibierung.

**Tabelle 2: Belagsinhibierung als Differenzmessung der Grauwerte**

| Formulierung | Grauwert (erfindungsgemäße Formulierung) - Grauwert(V-F.9) |
|---|---|
| F.1 | -0,3 |
| F.2 | -1,3 |
| F.3 | -0,6 |
| F.4 | -1,6 |
| F.5 | -3,5 |
| F.6 | -2,7 |
| F.7 | -2,0 |
| F.8 | -3,5 |

## Patentansprüche

1. Formulierung, enthaltend
(A) mindestens eine Verbindung, gewählt aus Methylglycindiacetat (MGDA) und Glutaminsäurediacetat (GLDA) sowie deren Salzen,
(B) mindestens ein Pfropfcopolymer, aufgebaut aus
(a) mindestens einer Pfropfgrundlage, gewählt aus nicht-ionischen Monosacchariden, Disacchariden, Oligosacchariden und Polysacchariden, und Seitenketten, erhältlich durch Aufpfropfen von
(b) mindestens einer ethylenisch ungesättigten Mono- oder Dicarbonsäure und
(c) mindestens einer Verbindung der allgemeinen Formel (I), 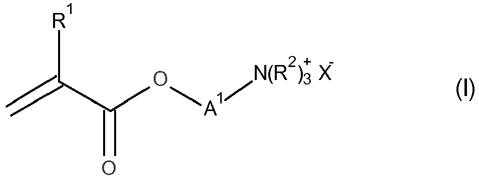
wobei die Variablen wie folgt definiert sind:
R¹ ist gewählt aus Methyl und Wasserstoff,
A¹ ist gewählt aus C₂-C₄-Alkylen,
R² sind gleich oder verschieden und gewählt aus C₁-C₄-Alkyl,
X- ist gewählt aus Halogenid, Mono-C₁-C₄-Alkylsulfat und Sulfat.

2. Formulierung nach Anspruch 1, **dadurch gekennzeichnet, dass** sie frei ist von Phosphaten und Polyphosphaten.

3. Formulierung nach Anspruch 1 oder 2, **dadurch gekennzeichnet, dass** man Verbindung (c) wählt aus ω-Trimethylaminoethyl(meth)acrylatochlorid.

4. Formulierung nach einem der Ansprüche 1 bis 3, **dadurch gekennzeichnet, dass** man Verbindung (A) wählt aus dem Trinatriumsalz von Methylglycindiacetat (MGDA).

5. Formulierung nach einem der Ansprüche 1 bis 4, **dadurch gekennzeichnet, dass** sie bei Zimmertemperatur fest ist.

6. Formulierung nach einem der Ansprüche 1 bis 5, **dadurch gekennzeichnet, dass** sie mindestens eine anorganische Peroxidverbindung (C) enthält.

7. Formulierung nach einem der Ansprüche 1 bis 6, **dadurch gekennzeichnet, dass** sie mindestens einen polymeren Builder (D) enthält.

8. Formulierung nach einem der Ansprüche 1 bis 7, **dadurch gekennzeichnet, dass** sie enthält:
insgesamt im Bereich von 1 bis 50 Gew.-% Verbindung (A),
insgesamt im Bereich von 0,1 bis 4 Gew.-% Pfropfcopolymer (B),
bezogen jeweils auf Feststoffgehalt der betreffenden Formulierung.

9. Verwendung von Formulierungen nach einem der Ansprüche 1 bis 8 zum Spülen von Geschirr und Küchenutensilien.

10. Verwendung von Formulierungen nach einem der Ansprüche 1 bis 8 zum Spülen von Gegenständen, die mindestens eine Oberfläche aus Glas aufweisen, welches dekoriert oder nicht dekoriert sein kann.

11. Verwendung nach Anspruch 7 oder 8, **dadurch gekennzeichnet, dass** es sich beim Spülen um ein Spülen mit einer Spülmaschine handelt.

12. Verfahren zur Herstellung von Formulierungen nach einem der Ansprüche 1 bis 8,
**dadurch gekennzeichnet, dass** man mindestens eine Verbindung (A) und mindestens ein Pfropfcopolymer (B) und gegebenenfalls einen oder mehrere weitere Inhaltsstoffe (D) und gegebenenfalls Peroxid (C) bzw. Chlorbleichmittel (C) in einem oder mehreren Schritten miteinander vermischt und anschließend gegebenenfalls Wasser ganz oder partiell entfernt.

13. Verfahren nach Anspruch 12, **dadurch gekennzeichnet, dass** man das Wasser durch Sprühtrocknung entfernt.

14. Pfropfcopolymer (B), aufgebaut aus
(a) mindestens einer Pfropfgrundlage, gewählt aus nicht-ionischen Monosacchariden, Disacchariden, Oligosacchariden und Polysacchariden,
und Seitenketten, erhältlich durch Aufpfropfen von
(b) mindestens einer ethylenisch ungesättigten Mono- oder Dicarbonsäure und
(c) mindestens einer Verbindung der allgemeinen Formel (I), 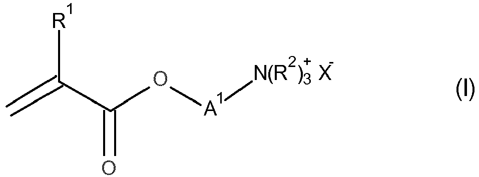 wobei die Variablen wie folgt definiert sind:
R¹ ist gewählt aus Methyl und Wasserstoff,
A¹ ist gewählt aus C₂-C₄-Alkylen,
R² sind gleich oder verschieden und gewählt aus C₁-C₄-Alkyl,
X⁻ ist gewählt aus Halogenid, Mono-C₁-C₄-Alkylsulfat und Sulfat.

15. Pfropfcopolymer (B) nach Anspruch 14, in der die Variablen wie folgt definiert sind:
R¹ ist Wasserstoff oder Methyl,
R² sind gleich und jeweils Methyl,
A¹ ist CH₂CH₂, und
X- ist Chlorid.

16. Verfahren zur Herstellung von Pfropfcopolymeren nach Anspruch 14 oder 15, **dadurch gekennzeichnet, dass** man
(b) mindestens eine ethylenisch ungesättigte Mono- oder Dicarbonsäure und
(c) mindestens eine Verbindung der allgemeinen Formel (I) in Gegenwart von mindestens einer Pfropfgrundlage (a)
radikalisch copolymerisiert.

## Claims

1. A formulation comprising
(A) at least one compound selected from methylglycine diacetate (MGDA) and glutamic acid diacetate (GLDA) and salts thereof,
(B) at least one graft copolymer composed of
(a) at least one graft base selected from nonionic monosaccharides, disaccharides, oligosaccharides and polysaccharides,
and side chains obtainable by grafting on of
(b) at least one ethylenically unsaturated mono- or dicarboxylic acid and
(c) at least one compound of the general formula (I), 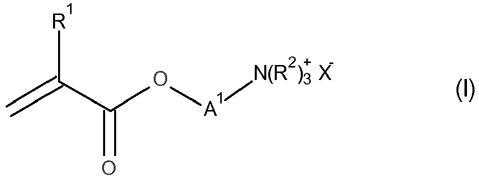
where the variables are defined as follows:
R¹ is selected from methyl and hydrogen,
A¹ is selected from C₂-C₄-alkylene,
R² are identical or different and selected from C₁-C₄-alkyl,
X⁻ is selected from halide, mono-C₁-C₄-alkyl sulfate and sulfate.

2. The formulation according to claim 1, wherein it is free from phosphates and polyphosphates.

3. The formulation according to claim 1 or 2, wherein compound (c) is selected from ω-trimethylamino-ethyl (meth)acrylatochloride.

4. The formulation according to any one of claims 1 to 3, wherein compound (A) is selected from the trisodium salt of methylglycine diacetate (MGDA).

5. The formulation according to any one of claims 1 to 4, wherein it is solid at room temperature.

6. The formulation according to any one of claims 1 to 5, wherein it comprises at least one inorganic peroxide compound (C).

7. The formulation according to any one of claims 1 to 6, wherein it comprises at least one polymeric builder (D).

8. The formulation according to any one of claims 1 to 7, wherein it comprises:
in total in the range from 1 to 50% by weight of compound (A),
in total in the range from 0.1 to 4% by weight of graft copolymer (B),
based in each case on solids content of the formulation in question.

9. The use of formulations according to any one of claims 1 to 8 for the washing of dishes and kitchen utensils.

10. The use of formulations according to any one of claims 1 to 8 for the washing of objects which have at least one surface made of glass, which may be decorated or undecorated.

11. The use according to claim 7 or 8, wherein the washing is washing using a dishwasher.

12. A process for the preparation of formulations according to any one of claims 1 to 8, wherein at least one compound (A) and at least one graft copolymer (B) and optionally one or more further ingredients (D) and optionally peroxide (C) or chlorine bleach (C) are mixed together in one or more steps and then optionally water is completely or partially removed.

13. The process according to claim 12, wherein the water is removed by spray drying.

14. A graft copolymer (B) composed of
(a) at least one graft base selected from nonionic monosaccharides, disaccharides, oligosaccharides and polysaccharides, and side chains obtainable by grafting on of
(b) at least one ethylenically unsaturated mono- or dicarboxylic acid and
(c) at least one compound of the general formula (I), 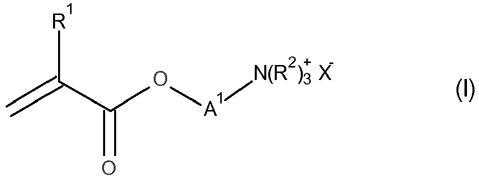 where the variables are defined as follows:
R¹ is selected from methyl and hydrogen,
A¹ is selected from C₂-C₄-alkylene,
R² are identical or different and selected from C₁-C₄-alkyl,
X⁻ is selected from halide, mono-C₁-C₄-alkyl sulfate and sulfate.

15. The graft copolymer (B) according to claim 14, in which the variables are defined as follows:
R¹ is hydrogen or methyl,
R² are identical and in each case methyl,
A¹ is CH₂CH₂, and
X⁻ is chloride.

16. A process for the preparation of graft copolymers according to claim 14 or 15,wherein
(b) at least one ethylenically unsaturated mono- or dicarboxylic acid and
(c) at least one compound of the general formula (I) are free-radically copolymerized in the presence of at least one graft base (a).

## Revendications

1. Formulation, contenant :
(A) au moins un composé choisi parmi le diacétate de méthylglycine (MGDA) et le diacétate de l'acide glutamique (GLDA), ainsi que leurs sels,
(B) au moins un copolymère greffé, formé par :
(a) au moins une base de greffage, choisie parmi les monosaccharides, les disaccharides, les oligosaccharides et les polysaccharides non ioniques,
et des chaînes latérales, pouvant être obtenues par greffage de
(b) au moins un acide mono- ou dicarboxylique éthyléniquement insaturé et
(c) au moins un composé de formule générale (I) 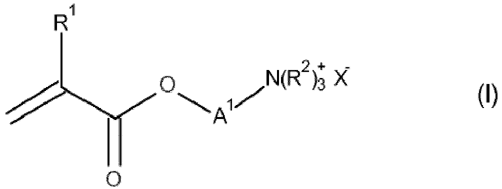 dans laquelle les variables sont définies de la manière suivante :
R¹ est choisi parmi méthyle et hydrogène,
A¹ est choisi parmi alkylène en C₂-C₄,
les R² sont identiques ou différents, et choisis parmi alkyle en C₁-C₄,
X⁻ est choisi parmi halogénure, mono-alkylsulfate en C₁-C₄ et sulfate.

2. Formulation selon la revendication 1, **caractérisée en ce qu'**elle est exempte de phosphates et de polyphosphates.

3. Formulation selon la revendication 1 ou 2, **caractérisée en ce que** le composé (c) est choisi parmi le (méth)acrylatochlorure d'ω-triméthylaminoéthyle.

4. Formulation selon l'une quelconque des revendications 1 à 3, **caractérisée en ce que** le composé (A) est choisi parmi le sel de trisodium de diacétate de méthylglycine (MGDA).

5. Formulation selon l'une quelconque des revendications 1 à 4, **caractérisée en ce qu'**elle est solide à température ambiante.

6. Formulation selon l'une quelconque des revendications 1 à 5, **caractérisée en ce qu'**elle contient au moins un composé de peroxyde inorganique (C).

7. Formulation selon l'une quelconque des revendications 1 à 6, **caractérisée en ce qu'**elle contient au moins un adjuvant polymère (D).

8. Formulation selon l'une quelconque des revendications 1 à 7, **caractérisée en ce qu'**elle contient :
au total dans la plage allant de 1 à 50 % en poids du composé (A),
au total dans la plage allant de 0,1 à 4 % en poids du copolymère greffé (B),
à chaque fois par rapport à la teneur en solides de la formulation en question.

9. Utilisation de formulations selon l'une quelconque des revendications 1 à 8 pour le lavage de la vaisselle et d'ustensiles de cuisine.

10. Utilisation de formulations selon l'une quelconque des revendications 1 à 8 pour le lavage d'articles qui comprennent au moins une surface en verre, qui peut être décorée ou non décorée.

11. Utilisation selon la revendication 7 ou 8, **caractérisée en ce que** le lavage est un lavage avec une machine à laver.

12. Procédé de fabrication de formulations selon l'une quelconque des revendications 1 à 8, **caractérisé en ce qu'**au moins un composé (A) et au moins un copolymère greffé (B) et éventuellement un ou plusieurs constituants supplémentaires (D) et éventuellement un peroxyde (C) ou un agent blanchissant chloré (C) sont mélangés les uns avec les autres en une ou plusieurs étapes, puis l'eau est éventuellement éliminée en totalité ou en partie.

13. Procédé selon la revendication 12, **caractérisé en ce que** l'eau est éliminée par séchage par pulvérisation.

14. Copolymère greffé (B), formé par :
(a) au moins une base de greffage, choisie parmi les monosaccharides, les disaccharides, les oligosaccharides et les polysaccharides non ioniques, et des chaînes latérales, pouvant être obtenues par greffage de
(b) au moins un acide mono- ou dicarboxylique éthyléniquement insaturé et
(c) au moins un composé de formule générale (I) 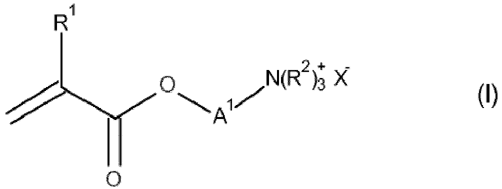 dans laquelle les variables sont définies de la manière suivante :
R¹ est choisi parmi méthyle et hydrogène,
A¹ est choisi parmi alkylène en C₂-C₄,
les R² sont identiques ou différents, et choisis parmi alkyle en C₁-C₄,
X⁻ est choisi parmi halogénure, mono-alkylsulfate en C₁-C₄ et sulfate.

15. Copolymère greffé (B) selon la revendication 14, dans lequel les variables sont définies de la manière suivante :
R¹ est hydrogène ou méthyle,
les R² sont identiques, et signifient chacun méthyle,
A¹ signifie CH₂CH₂, et
X⁻ signifie chlorure.

16. Procédé de fabrication de copolymères greffés selon la revendication 14 ou 15, **caractérisé en ce que**
(b) au moins un acide mono- ou dicarboxylique éthyléniquement insaturé, et
(c) au moins un composé de formule générale (I) sont copolymérisés par voie radicalaire en présence d'au moins une base de greffage (a).
